(19) Europäisches Patentamt
European Patent Office
Office européen des brevets

(11) EP 0 998 308 B1

(12) EUROPEAN PATENT SPECIFICATION

(45) Date of publication and mention of the grant of the patent:
**19.12.2007 Bulletin 2007/51**

(21) Application number: **98932332.4**

(22) Date of filing: **01.07.1998**

(51) Int Cl.:
***A61K 49/00*** *(2006.01)*

(86) International application number:
**PCT/GB1998/001931**

(87) International publication number:
**WO 1999/001162 (14.01.1999 Gazette 1999/02)**

# (54) DIAGNOSTIC METHOD FOR THE DETECTION OF MIOCARDIAL ISCHEMIA

DIAGNOSTISCHES VERFAHREN ZUM NACHWEIS VON MYOKARD-ISCHÄMIE

METHODE DIAGNOSTIQUE POUR DETECTER UNE ISCHEMIE DU MYOCARDE

(84) Designated Contracting States:
**AT BE CH CY DE DK ES FI FR GB GR IE IT LI LU MC NL PT SE**

(30) Priority: **01.07.1997 GB 9713963**
**25.07.1997 US 53837 P**

(43) Date of publication of application:
**10.05.2000 Bulletin 2000/19**

(73) Proprietors:
- **GE Healthcare AS**
  **0485 Oslo (NO)**
- **UNIVERSITY OF CALIFORNIA, SAN FRANCISCO**
  **San Francisco, CA 94143 (US)**

(72) Inventors:
- **WATSON, Alan**
  **Lexington, MA 02420 (US)**
- **WENDLAND, Michael,**
  **Benicia, CA 94510 (US)**
- **JYNGE, Per**
  **N-7030 Trondheim (NO)**
- **KARLSSON, Jan, Olof**
  **N-7038 Trondheim (GB)**
- **BRUROK, Heidi**
  **N-7038 Trondheim (NO)**
- **RONGVED, P I**
  **N-1450 Nesoddtangen (NO)**
- **SAEED, Maythem,**
  **Novato CA 94945 (US)**

(74) Representative: **Flechsler, Insa**
**GE Healthcare Limited**
**Amersham Place**
**Little Chalfont,**
**Bucks. HP7 9NA (GB)**

(56) References cited:
**EP-A- 0 290 047**

- **EDELMAN R R ET AL: "Contrast-enhanced echo - planar MR imaging of myocardial perfusion: preliminary study in humans." RADIOLOGY, (1994 MAR) 190 (3) 771-7. JOURNAL CODE: QSH. ISSN: 0033-8419., XP002083542 United States**
- **WENDLAND M F ET AL: "Inversion recovery EPI of bolus transit in rat myocardium using intravascular and extravascular gadolinium-based MR contrast media: Dose effects on peak signal enhancement." MAGNETIC RESONANCE IN MEDICINE 32 (3). 1994. 319-329. ISSN: 0740-3194, XP002083543**
- **WENDLAND M.F. ET AL: "Echo - planar MR imaging of normal and ischemic myocardium with gadodiamide injection." RADIOLOGY, (1993) 186/2 (535-542). ISSN: 0033-8419 CODEN: RADLAX, XP002083544 United States**
- **YU K.K. ET AL: "Real-time dynamics of an extravascular magnetic resonance contrast medium in acutely infarcted myocardium using inversion recovery and gradient- recalled echo - planar imaging." INVEST. RADIOL., (1992) 27/11 (927-934). ISSN: 0020-9996 CODEN: INVRAV, XP002083545 United States**
- **POMEROY O H ET AL: "Magnetic resonance imaging of acute myocardial ischemia using a manganese chelate, Mn-DPDP." INVESTIGATIVE RADIOLOGY, (1989 JUL) 24 (7) 531-6. JOURNAL CODE: GWK. ISSN: 0020-9996., XP002083546 United States**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- **SAEED M ET AL: "OCCLUSIVE AND REPERFUSED MYOCARDIAL INFARCTS: DIFFERENTIATION WITH MN-DPDP-ENHANCED MR IMAGING 1" RADIOLOGY, vol. 172, no. 1, July 1989, pages 59-64, XP002043393**
- **SAEED M ET AL: "Reversible and irreversible injury in the reperfused myocardium: differentiation with contrast material-enhanced MR imaging." RADIOLOGY, (1990 JUN) 175 (3) 633-7. JOURNAL CODE: QSH. ISSN: 0033-8419., XP002083547 United States**
- **NELSON R. C. ET AL.: "Manganese Dipyridoxyl Diphosphate. Effect on dose, time, and pulse sequence on the hepatic enhancement in rats" INVESTIGATIVE RADIOLOGY, XP002083548**

## Description

The present invention relates to improvements in and relating to magnetic resonance imaging, in particular to a method of MR imaging enabling early detection of myocardial ischemia.

Ischemia-related diseases, in particular coronary artery diseases, account for the majority of deaths in Western countries. Myocardial ischemia is a serious condition and any delay in reperfusion of the ischemic tissues can be life-threatening. Rapid identification and location of myocardial ischemia is therefore highly desirable so that the necessary action, be it therapeutic or surgical, can be taken promptly before irreversible myocardial damage occurs.

Ischemic injury can be considered to result from two main events: (i) hypoxia leading to an inadequate supply of oxygen to the tissues; and (ii) decreased transport of metabolic substrates to the tissues and of metabolic end products from the tissues. Immediate consequences include energy deficit and an accumulation of protons and lactate in the region of ischemia. Other consequences include a marked, potentially harmful stimulation of the sympathetic nervous system which ultimately leads to a rapid loss of adenosine triphosphate (ATP), an early onset of acidosis and decreased organ function.

Cardiac tissue, like other metabolically active tissues, are particularly vulnerable to ischemic injury. The initial phase of acute myocardial infarction is in general associated with a loss of normal contractile function which manifests itself as regional dyskinesia. This may be due to an,abrupt fall in coronary perfusion pressure which induces an acute hibernating state and to the rapid cessation of normal transmembrane ion transport. Reperfusion of the ischemic myocardium prior to the onset of irreversible injury may lead to a rapid return or a delayed return (stunning) to normal cardiac metabolism and function.

Magnetic resonance imaging (MRI) has been established as a useful cardiac imaging technique. Although MR techniques using spin-echo imaging are capable of showing the anatomy of the heart, the use of contrast agents is necessary for the detection of myocardial ischemia and infarction.

To date, only paramagnetic substances have been investigated for use as myocardial contrast agents. The metal ions manganese and gadolinium have received the most attention. Although the use of free manganese and gadolinium ions in the body is limited by their toxicities, complexing of these cations with a variety of ligands serves to greatly reduce their toxicity whilst retaining their paramagnetic properties.

Gd-DTPA has been the subject of extensive clinical testing for use in myocardial MR imaging. Although this metal complex has been shown to improve identification of acute myocardial infarcts on MR images in animals and humans, its rapid elimination by renal excretion and its distribution within the extracellular fluid space severely limits its clinical use in imaging of the myocardium.

While Gd-DTPA distributes into the extracellular fluid space, manganese enters the intracellular space since it has access to the cardiomyocytes via slow $Ca^{2+}$ channels. It is also strongly paramagnetic, resulting in a dramatic $T_1$ shortening and thus increased signal intensity in normal myocardial tissue. Manganese ions are actively taken up by viable myocardial cells and have a short half-life in the blood pool resulting in a high myocardial to blood ratio. However, during coronary occlusion, blood flow to myocardial tissue is greatly restricted, thereby reducing exposure of myocardial cells to manganese ions which in turn greatly retards manganese uptake. Uptake of manganese is, however, resumed in viable cells following reperfusion. Such properties can be used in MRI diagnosis of severe coronary heart disease. Indeed, several proposals have been made for the use of MnDPDP in $T_1$-weighted spin echo imaging for detection of acute myocardial infarction (see Pomeroy et al., Invest. Radiology 24:531-536, 1989; Brown et al., AJR 151:865-872, 1988; Saeed et al., Radiology 172:59-64, 1989; and Saeed et al., Radiology 175:633-637, 1990).

However, administered by rapid bolus injection at high dosages, manganese can interfere with normal functioning of the heart. As a result, bolus injection of low doses and infusion of low or high dosages of manganese may be contemplated for clinical use. Use of conventional $T_1$-weighted imaging procedures, such as described in the prior art (supra) requires intravenous injection of relatively large doses (0.2 to 0.4 mmol/kg) of manganese to produce adequate contrast between normal and ischemic or infarcted myocardium. Such doses greatly exceed that proposed for clinical use. The use of manganese for in vivo MR imaging of the myocardium therefore remains problematical and there still exists a need for a clinically acceptable method of MR imaging capable of providing information about the cellular function of the myocardium, in particular a method capable of providing sufficient contrast between normal and ischemic tissue without further damaging the heart.

In the studies described above, myocardial ischemia was induced by coronary ligation, i.e. by purely physical means which prevented injected or infused manganese from reaching non-perfused tissues. However, it has now surprisingly been found that the cellular process of manganese uptake is greatly retarded during early ischemia thereby providing for the possibility of using manganese contrast agents in a method of functional myocardial imaging.

It has now surprisingly been found that substantially lower, clinically acceptable, dosages of manganese may be used in fast or ultra-fast imaging techniques to provide an effective method of myocardial imaging, in particular to provide important information about myocardial viability during or following a severe heart attack or coronary occlusion. Viewed from one aspect the invention provides the use of a physiologically acceptable manganese complex or salt

thereof for the manufacture of a contrast medium for use in a method of detecting myocardial ischemia in a human or non-human, especially mammalian, body, said method comprising administering said complex or salt thereof to said body at a dosage of 0.001 to 0.2 mmol/kg bodyweight, subjecting said body to an inversion recovery echo planar imaging procedure capable of generating images with time intervals of less than 0.5 seconds and thereafter providing a series of images of the myocardium of said body whereby to identify regions of abnormal blood flow.

Thus, the invention provides for the detection of any blood flow abnormalities in the myocardium, e.g. regions of blood flow deficit or increase. The method of the invention is particularly suited to the early detection of myocardial ischemia and permits detection of ischemia substantially less than 1 hour after occurrence, e.g. after about 30 minutes, as opposed to the 2-3 hours required by more conventional techniques.

In this way, reperfusion of ischemic tissue is possible at an early stage thus substantially reducing the chance of permanent tissue damage.

Whilst the determination of the existence and location of ischemic areas is important, it is also highly desirable to be able to detect the degree or severity of the ischemia and to discriminate such from permanently infarcted tissue. This too can be achieved according to the invention.

Viewed from a further aspect, the invention thus provides the use of a physiologically acceptable manganese complex or salt thereof for the manufacture of a contrast medium for use in a method of evaluating the severity of myocardial ischemia in a human or non-human, especially mammalian, body, said method comprising administering said complex or salt thereof to said body at a dosage of 0.001 to 0.2 mmol/kg bodyweight, subjecting said body to an inversion recovery echo planar imaging procedure capable of generating images with time intervals of less than 0.5 seconds and thereafter providing a series of images of the myocardium of said body whereby to indicate the degree of blood perfusion deficit in the myocardium.

Preferably, the invention provides for functional imaging which may discriminate between normal tissue, reversibly and irreversibly injured tissue during ischemia and during reperfusion. In particular, the invention provides a means for discriminating between reversibly and irreversibly injured tissue.

Thus, viewed from a yet further aspect the invention provides the use of a physiologically acceptable manganese complex or salt thereof for the manufacture of a contrast medium for use in a method of discriminating between reversibly and irreversibly injured myocardial tissue in a human or non-human body, said method comprising administering said complex or salt thereof to said body at a dosage of 0.001 to 0.2 mmol/kg bodyweight, subjecting said body to an inversion recovery echo planar imaging procedure capable of generating images with time intervals of less than 0.5 seconds and thereafter providing a series of images of the myocardium of said body whereby to discriminate reversibly from irreversibly injured tissue.

The invention relates to methods preferably carried out using highly $T_1$-sensitive, fast or ultra-fast imaging techniques which enable the generation of a series of images with a short a time interval as possible between successive images. This ensures the acquisition of multiple images during the first passage of the contrast medium through the heart, thus enabling a clinically acceptable dose of contrast medium to be used. Techniques capable of generating images with time intervals of less than 100 milliseconds, e.g. from 20 to 80 milliseconds are particularly preferred. Thus, the invention relates to inversion recovery echo planar imaging, e.g. gradient refocused inversion recovery echo planar imaging. Particularly suitable echo planar imaging techniques are those in which TI (inversion time) is 100 to 800 msecs, e.g. 700 msecs, TR (repetition time) is 2000 msecs and TE (echo time) is less than 20 msecs, e.g. 10-20 msecs. The sensitivity of the imaging technique may be increased by gating to every heart beat.

Flip angles for use in the preparation interval preceding image acquisition may be either 180° or 90°, although 90° is preferred. If a 90° flip angle is used then TI may conveniently be in the range of 200 to 500 msecs. If a 180° flip angle is used then TI should preferably be at least 600 msecs for all intensity values ≥0. TR is determined by the subject's heart rate. Using a 90° flip angle it is preferable to acquire single heart beat temporal resolution.

In accordance with the invention, an indication of the degree of blood flow for a given ROI (region of interest) may readily be determined by comparing the MR signal intensity for that ROI with a reference value, e.g. the signal intensity for similar tissue known to have normal blood flow. Such reference values may be predetermined or may be selected as the MR signal intensity values for ROIs in normal tissue in the same image. Thus, for example, signal intensity measurements may be obtained at several positions on each image, e.g. interventricular septum (representing normal myocardium), anterolateral wall (representing reversibly or irreversibly injured myocardium), skeletal muscle and oil phantom (included as a standard reference for intensity), with all signal intensity values being standardised to the signal intensity of the oil phantom. The position of the region of interest then remains fixed for analysis of subsequent images. The contrast between normal and injured regions may then be expressed as the ratio of the signal intensity of infarcted tissue to that of normal myocardial tissue. As is discussed more fully in the accompanying examples, the location and extent of regions having reduced blood flow detected according to the invention corresponds closely to the location and extent as determined using conventional non-MRI techniques such as histopathological tissue staining.

As outlined above, the invention provides for the use of much lower, clinically acceptable, doses of manganese. The manganese contrast agent is administered at a dosage of from 0.001 to 0.2 mmol/kg body weight, preferably from

0.005 to 0.2 mmol/kg body weight. More preferably, the dosage of manganese will be from 0.01 to 0.05 mmol/kg body weight, e.g. 0.03 to 0.05 mmol/kg body weight. Preferably, the contrast agent will be administered by bolus injection or infusion into the systemic vasculature (e.g. 3 to 5 ml/kg/minute).

Besides its application in terms of identifying and providing an indication of the severity of acute myocardial ischemias or infarcts, the present invention has a broad range of possible applications, including the following:

- delineation of the area at risk of infarction after acute coronary artery occlusion;
- differentiation between occlusive and reperfused myocardial infarctions;
- discrimination between successfully reperfused stunned (viable, brief coronary occlusion followed by reperfusion) and reperfused infarcted (necrotic, long coronary occlusion followed by reperfusion) tissues;
- differentiation between reversible and irreversible myocardial injury.

The invention is capable of distinguishing between areas of normal blood flow, reduced blood flow and no blood flow, and may also be used to monitor reperfusion of ischemic tissue, e.g. following acute coronary interventions in the form of fibrinolysis and PTCA. Viewed from a still further aspect, the invention thus provides Use of a physiologically acceptable manganese complex or salt thereof for the manufacture of a contrast medium for use in a method of monitoring reperfusion of the myocardium of a human or non-human, especially mammalian, body, said method comprising administering said complex or salt thereof to said body at a dosage of 0.001 to 0.2 mmol/kg bodyweight, subjecting said body to an inversion recovery echo planar imaging procedure capable of generating images with time intervals of less than 0.5 seconds and thereafter providing a series of images of the myocardium of said body whereby to identify regions of reperfusion.

Provided the ischemic area is effectively reperfused prior to onset of irreversible tissue damage, manganese uptake and contrast enhancement returns to normal. The rise in contrast is maintained over time since manganese ions are, in general, retained intracellularly in the myocardial cells. If on the other hand the area at risk has become necrotic, cellular leakage leads to extravasation and tissue washout of the contrast agent. If this is the case then the manganese-induced rise in contrast will not be maintained over time as the manganese ions are more rapidly washed out of the necrotic zone.

Conveniently, magnetic resonance imaging is carried out within a period of up to 6 hours following injection of the manganese contrast agent. However, delayed imaging techniques in which imaging is carried out within a period of from 3 to 6 hours, e.g. about 4 hours post injection have been found to be particularly effective in distinguishing infarcted from normal myocardium and in characterising the severity of damage in the injured zone. Whilst not wishing to be bound by theoretical considerations, manganese is believed to be rapidly taken up by viable myocardial cells and retained, whereas in reperfused infarcted tissue manganese is believed to rapidly distribute throughout the tissue but is not retained by non-viable cells and is efficiently cleared from the tissue albeit more slowly than it is cleared from the blood. As a result, early after reperfusion the infarction zone has a high signal intensity due to an enlarged myocardial distribution volume providing relatively poor contrast between normal and reperfused infarcted myocardium. A sufficient delay in imaging following administration of the contrast agent thus ensures that the agent has cleared from non-viable cells but not from viable tissue from which clearance of the contrast agent is much less rapid.

Viewed from a yet further aspect the invention thus provides Use of a physiologically acceptable manganese complex or salt thereof for the manufacture of a contrast medium for use in a method of distinguishing viable myocardial tissue from necrotic (infarcted) tissue in a human or non-human body, said method comprising administering said complex or salt thereof to said body at a dosage of 0.001 to 0.2 mmol/kg bodyweight, subjecting said body to an inversion recovery echo planar imaging procedure capable of generating images with time intervals of less than 0.5 seconds and thereafter providing a series of images of the myocardium of said body whereby to distinguish viable myocardial tissue from infarcted tissue.

The return of normal cardiac function following reversible ischemia leads to normalisation of both cellular uptake and retention of manganese. Delayed imaging can thus be used to identify viable myocardium after reopening of coronary arteries by PTCA or by fibrinolytic therapy.

Viewed from a still further aspect, the invention provides a method of imaging the myocardium in a human or non-human body previously administered with a physiologically acceptable manganese complex or salt thereof at a dosage of 0.001 to 0.2 mmol/kg bodyweight, said method comprising subjecting said body to an inversion recovery echo planar imaging procedure capable of generating images with time intervals of less than 0.5 seconds and thereafter providing a series of images of the myocardium of said body.

In addition to providing important information about myocardial viability, the manganese ions ($Mn^{2+}$) used according to the invention are also believed to act as oxygen radical scavengers, thereby serving to protect the ischemic heart from further tissue damage which can occur during reperfusion. The manganese ions also serve to prolong the biological activity of nitric oxide generated in intact endothelial cells, thereby facilitating vasodilation.

The manganese contrast agent to be used in the invention should preferably be an intravascular contrast agent,

i.e. one which is substantially retained within the systemic vasculature at least until it has passed to the heart.

The manganese contrast agent may be in the form of an ionic or more preferably a non-ionic complex. Especially preferred are manganese chelate complexes, which may be bound to one or more carrier molecules.

Preferred manganese chelate complexes are those which dissociate *in vivo* to provide a release of manganese ions on passage through the heart. Conveniently, the manganese chelate may have a Ka value in the range of from $10^7$ to $10^{25}$, more preferably $10^9$ to $10^{24}$, yet more preferably $10^{10}$ to $10^{23}$, e.g. $10^{12}$ to $10^{22}$.

A wide range of suitable chelants and macromolecule bound chelants for manganese ions have been proposed. Particularly suitable chelants for the formation of the manganese contrast agents for use in the method of the invention include the macrocyclic and more preferably the linear or branched polyaminopolycarboxylic acid or carboxylic acid derivatives described in EP-A-299795, EP-A-71564, DE-A-3401052, EP-A-203962 and EP-A-436579 and the phosphorus oxyacid analogs. Preferred chelants include N,N,N',N",N"-diethylenetriaminepentaacetic acid (DTPA) and 6-carboxymethyl-3,9-bis(methylcarbamoylmethyl)-3,6,9-triazaundecanedioic acid (DTPA-BMA).

Dipyridoxyl based chelating agents have also been described for use as MRI contrast agents, for example PLED (N,N'-dipyridoxyl ethylenediamine-N,N'-diacetic acid) derivatives and analogues. Manganese (II) chelates with such chelating agents are particularly preferred for use in the invention.

A possible cause of tissue damage during reperfusion is believed to be the availability of ferric ions which catalyse the formation of free radicals. Chelating agents, such as the dipyridoxyl based chelating agents described above, which are capable of effectively binding ferric ions are thus preferred for use in the method of the invention. Those chelating agents which preferentially bind to ferric ions serve to enhance the oxygen radical defence in part already provided by the manganese ions. In this regard, preferred manganese chelates are those having a Ka value smaller by a factor of at least $10^3$ than the Ka value of the corresponding ferric ($Fe^{3+}$) chelate.

Preferred for use according to the invention are manganese chelates of a compound of formula I and salts thereof

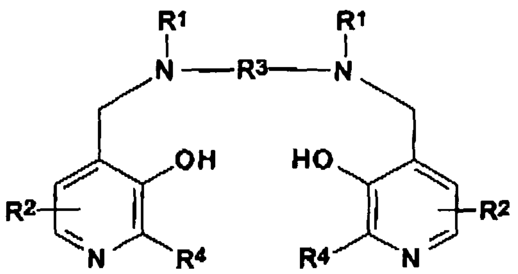

(I)

(wherein in formula I

each $R^1$ independently represents hydrogen or $-CH_2COR^5$ ;
$R^5$ represents hydroxy, optionally hydroxylated alkoxy, amino or alkylamido;
each $R^2$ independently represents a group $XYR^6$;
X represents a bond, or a $C_{1-3}$ alkylene or oxoalkylene group optionally substituted by a group $R^7$;
Y represents a bond, an oxygen atom or a group $NR^6$;
$R^6$ is a hydrogen atom, a group $COOR^8$, an alkyl, alkenyl, cycloalkyl, aryl or aralkyl group optionally substituted by one or more groups selected from $COOR^8$, $CONR^8{}_2$, $NR^8{}_2$, $OR^8$, $=NR^8$, =0, OP (O) $(OR^8)$ $R^7$ and $OSO_3M$;
$R^7$ is hydroxy, an optionally hydroxylated, optionally alkoxylated alkyl or aminoalkyl group;
$R^8$ is a hydrogen atom or an optionally hydroxylated, optionally alkoxylated alkyl group;
M is a hydrogen atom or one equivalent of a physiologically tolerable cation, e.g. an alkali or alkaline earth cation, an ammonium ion or an organic amine cation, such as a meglumine ion;
$R^3$ represents a $C_{1-8}$ alkylene group, preferably a $C_{1-6}$, e.g. a $C_{2-4}$ alkylene group, a 1,2-cycloalkylene group, or a 1,2-arylene group; and
each $R^4$ independently represents hydrogen or $C_{1-3}$ alkyl).

As used herein the terms "alkyl" and "alkylene" include both straight-chained and branched, saturated and unsaturated hydrocarbons. The term "1,2-cycloalkylene" includes both cis and trans cycloalkylene groups and alkyl substituted cycloalkylene groups having from 5-8 carbon atoms. The term "1,2-arylene" includes phenyl and napthyl groups and alkyl substituted derivatives thereof having from 6 to 10 carbon atoms.

Unless otherwise specified, any alkyl, alkylene or alkenyl moiety may conveniently contain from 1 to 20, preferably 1-8, more preferably 1-6 and especially preferably 1-4 carbon atoms.

Cycloalkyl, aryl and aralkyl moieties may conveniently contain 3-18, preferably 5-12 and especially preferably 5-8 ring atoms. Aryl moieties comprising phenyl or naphthyl groups are preferred. As aralkyl groups, phenyl $C_{1-3}$ alkyl, especially benzyl, are preferred.

Where groups may optionally be substituted by hydroxy groups, this may be monosubstitution or polysubstitution and, in the case of polysubstitution, alkoxy and/or hydroxy substituents may be carried by alkoxy substituents.

In formula I, $R^5$ is preferably hydroxy, $C_{1-8}$ alkoxy, ethylene glycol, glycerol, amino or $C_{1-8}$ alkylamido. Preferably each group $R^1$ represents $-CH_2COR^5$ in which $R^5$ is hydroxy.

In the compounds of formula I, X is preferably a bond or a group selected from $CH_2$, $(CH_2)_2$, CO, $CH_2CO$, $CH_2CH_2CO$ or $CH_2COCH_2$.

The compounds of formula I may have the same or different $R^2$ groups on the two pyridyl rings and these may be attached at the same or different ring positions. However, it is especially preferred that substitution be at the 5- and 6-positions, most especially the 6-position, i.e. para to the hydroxy group. Compounds in which the $R^2$ groups are identical and identically located, e.g. 6,6', are especially preferred.

Preferred as groups $R^6$ are mono- or poly(hydroxy or alkoxylated) alkyl groups.

$R^7$ is preferably an unsubstituted alkyl or aminoalkyl group.

Particularly preferred identities for group $R^2$ include $CHR^7OCO(CH_2)_xPh$ and $CHR^7OCO(CH_2CO)_xPh$ (wherein x is 1 to 3), $CHR^7OCOBu^t$, $CH_2N(H)R^{6'}$, $CH_2N(R^{6'})_2$, $N(H)R^{6'}$, $N(R^{6'})_2$, $CH_2OH$, $CH_2OR^{6'}$, $COOR^{6'}$, $CON(H)R^{6'}$, $CON(R^{6'})_2$ or $OR^{6'}$ (where $R^{6'}$ is a mono- or polyhydroxylated, preferably $C_{1-4}$, especially preferably $C_{1-3}$, alkyl group), $(CH_2)_nCOOR^{7'}$ (wherein n is 1 to 6), $COOR^{7'}$ (where $R^{7'}$ is a $C_{1-4}$ alkyl, preferably $C_{1-3}$, especially preferably a methyl group), $CH_2OSO_3^-M$, $CH_2CH_2COOH$, $CH_2OP(O)(OH)(CH_2)_3NH_2$, $CH_2OP(O)(OH)CH_3$ or $CH_2OP(O)(OH)_2$ group.

Compounds of formula I in which $R^3$ is ethylene and $R^2$ has any of the identities listed above are particularly preferred.

Especially preferred for use in the invention is the manganese (II) chelate of N,N'-bis-(pyridoxal-5-phosphate)-ethylenediamine-N,N'-diacetic acid (MnDPDP). Also preferred for use in the invention is the manganese (II) chelate of N,N'-dipyridoxyl-ethylenediamine-N,N'-diacetic acid (MnPLED). If not all of the labile hydrogens of the chelates are substituted by the complexed metal ion, biotolerability and/or solubility of the chelate may be increased by substituting the remaining labile hydrogen atoms with physiologically biocompatible cations of inorganic and/or organic bases or amino acids. Examples of suitable inorganic cations include $Li^+$, $K^+$, $Na^+$ and especially $Ca^{2+}$. Suitable organic cations include ammonium, substituted ammonium, ethanolamine, diethanolamine, morpholine, glucamine, N,N,-dimethyl glucamine, lysine, arginine or ornithine.

The compounds for use in the invention may be prepared by procedures known in the art. Suitable methods for preparing the polyaminopolycarboxylic acid based chelating agents are described in EP-A-299795, EP-A-71564, DE-A-3401052, EP-A-203962 and EP-A-436579.

In preparing the dipyridoxyl compounds, the compound PLED may be used as a starting material and may be appropriately derivatised using conventional procedures to obtain the compounds of formula I. Suitable methods for preparing the compounds of formula I are described for example in EP-A-290047.

Alternatively the compounds of formula I may be prepared by reacting the corresponding pyridoxal compound with an alkylene diamine according to the procedure for making PLED described by Taliaferro (Inorg. Chem. 23: 1183-1192, 1984).

The manganese chelates for use in accordance with the invention may be formed by conventional procedures known in the art. In general, such processes involve dissolving or suspending a metal oxide or metal salt (e.g. nitrate, chloride or sulfate) in water or a lower alcohol such as methanol, ethanol, or isopropanol. To this solution or suspension is added an equimolar amount of the chelating agent in water or a lower alcohol and the mixture is stirred, if necessary with heating moderately or to the boiling point, until the reaction is completed. If the chelate salt formed is insoluble in the solvent used, the reaction product is isolated by filtering, If it is soluble, the reaction product is isolated by evaporating to dryness, e.g. by spray drying or lyophilising.

If acid groups such as the phosphoric acid groups are still present in the resulting chelate, it is advantageous to convert the acidic chelate salt into a neutral chelate salt by reaction with inorganic and/or organic bases or amino acids, which form physiologically acceptable cations, and to isolate them.

The carboxylic and phosphoric acid groups of the chelating agents can also be neutralised by esterification to prepare carboxylate and phosphate esters. Such esters can be prepared from the corresponding alcohols by conventional procedures known in the art. Suitable esters include, for example, esters of straight-chained or branched alcohols having from 1 to 18 carbon atoms, mono and polyhydric alkyl amino alcohols having from 1 to 18 carbon atoms, preferably having from 1 to 6 carbons, such as serinol or diethanolamine, and polyhydric alcohols having from 1 to 18 carbon atoms, such as ethylene glycol or glycerol.

Where the metal chelate carries an overall charge it will conveniently be used in the form of a salt with a physiologically acceptable counterion, for example an ammonium, substituted ammonium, alkali metal or alkaline earth metal (e.g. calcium) cation or an anion deriving from an inorganic or organic acid. In this regard, meglumine salts are

particularly preferred.

According to the invention, images of the myocardium are preferably generated during the early stages following the ischemia inducing event, e.g. within two days, preferably within 1 day, especially preferably within 12 hours and more especially preferably within 6 hours of ischemia occurring. In this event, the invention can advantageously be used to follow and determine the success of intervention to cause reperfusion.

For use in the invention, the contrast agents may be formulated with conventional pharmaceutical or veterinary formulation aids, for example stabilizers, antioxidants, osmolality adjusting agents, buffers, pH adjusting agents, etc. and may be in a form suitable for injection or infusion directly or after dispersion in or dilution with a physiologically acceptable carrier medium, e.g. water for injections. Thus the contrast agents may be in a conventional pharmaceutical administration form such as a powder, solution, suspension, dispersion, etc. However, solutions, suspensions and dispersions in physiologically acceptable carrier media will generally be preferred.

The contrast agents may therefore be formulated for administration using physiologically acceptable carriers or excipients in a manner well-known to those skilled in the art. For example, the compounds, optionally with the addition of pharmaceutically acceptable excipients, may be suspended or dissolved in an aqueous medium, with the resulting solution or suspension then being sterilized. Suitable additives include, for example, physiologically biocompatible buffers (e.g. DTPA or DTPA-bisamide) or calcium chelate complexes (e.g. calcium DTPA salts, calcium DTPA-bisamide salts or NaCaDTPA-bisamide) or, optionally, additions (e.g. 1 to 50 mole percent) of calcium or sodium salts (e.g. calcium chloride, calcium ascorbate, calcium gluconate or calcium lactate).

For effective uptake by the calcium channels in the cardiomycytes, the manganese should be in the state $Mn^{2+}$.

To inhibit oxidation to $Mn^{3+}$, the compositions used in the methods of the invention will preferably contain an antioxidant, e.g. ascorbic acid or a reducing sugar.

For mr imaging, manganese will preferably be administered at a dose of 0.001 to 0.2 mmol/kg preferably 0.01 to 0.05 mmol/kg.

Parenterally administrable forms, e.g. intravenous solutions, should be sterile and free from physiologically unacceptable agents, and should have low osmolality to minimize irritation or other adverse effects upon administration, and thus the contrast medium should preferably be isotonic or slightly hypertonic. Suitable vehicles include aqueous vehicles customarily used for administering parenteral solutions such as Sodium Chloride Injection, Ringer's Injection, Dextrose Injection, Dextrose and Sodium Chloride Injection, Lactated Ringer's Injection and other solutions such as are described in Remington's Pharmaceutical Sciences, 15th ed., Easton: Mack Publishing Co., pp. 1405-1412 and 1461-1487 (1975) and The National Formulary XIV, 14th ed. Washington: American Pharmaceutical Association (1975). The solutions may contain preservatives, antimicrobial agents, buffers and antioxidants conventionally used for parenteral solutions, excipients and other additives which are compatible with the contrast agents and which will not interfere with the manufacture, storage or use of the products.

The invention will now be illustrated further by way of example with particular reference to certain nonlimiting embodiments and to the accompanying figures,

Study 1

The aim of the study was to examine the potential of MnDPDP for delineating acute regional ischemia in rats by comparing dose-dependent myocardial enhancement during bolus passage of low doses of MnDPDP.

MATERIALS AND METHODS:

Sprague-Dawley rats (250-300g) were anaesthetised with 50 mg/kg sodium pentobarbital and mechanically ventilated after tracheostomy. For bolus injection of the contrast agent, a catheter was placed in the femoral vein. In order to produce regional ischemia, left thoracotomy was performed followed by occlusion of the anterior branch of the left coronary artery. The potential of various doses of MnDPDP in demarcating regional ischemia was determined. Four groups of animals were designed, each group (n = 7-8 rats) received one bolus dose (1 ml/kg) of MnDPDP (0.005, 0.010, 0.020, or 0.040 mmol/kg). MR imaging was performed between 15-20 mins after acute coronary occlusion.

ECG-gated inversion recovery echoplanar images were acquired using a GE Omega 2.0 T system and a home-built points to define a field-of-volume (FOV) of 40x40mm was used. TE was set to 10 msec by acquiring an asymmetric echo. Inversion recovery was accomplished using a composite non-selective inversion pulse which was carefully optimized prior to administration of the contrast medium. Shortly after the occlusion of the coronary vessel (15-25 mins), IR EP imaging was used to monitor bolus passage of MnDPDP through the regionally ischemic heart. Fully relaxed $T_1$-sensitive inversion recovery EP images (TR = 5s) were acquired in a set of 16 images. Then, 32 inversion- recovery echoplanar images every 2 secs were obtained with inversion time of 700msecs (to nullify myocardial signal) to monitor the dynamic $T_1$ changes in myocardial signal intensity during the passage of the contrast medium. Changes in myocardial signal as a function of time were plotted. ROIs were obtained from non-ischemic and ischemic myocardium as well as from LV

chamber blood. ROIs were determined on images acquired post-contrast injection, where the ischemic region is clearly defined. Alterations in signal intensity were referred to that of fully relaxed intensity (=100%).

At the conclusion of the imaging protocol, the presence of coronary occlusion and acute ischemia was confirmed at postmortem by injecting 0.5 ml/kg phthalocyanine blue dye intravenously. Blue dye was then allowed to circulate for 3 mins before the heart was excised. The dye stains normally perfused myocardium blue, but does not stain the ischemic region.

RESULTS:

Figure 1 shows non-selective IR GRE images obtained during the transit of 0.040 mmol/kg MnDPDP in a rat subjected to acute occlusion of the coronary artery (~ 10 min). The images shown were acquired before contrast agent was injected (top left), as the bolus entered the right ventricular (RV) chamber (top centre), as the bolus entered the left ventricular (LV) chamber (top right), at the point of maximal blood signal (bottom left) and maximal increase in myocardial signal (bottom centre), and the final image acquired in set. The ischemic region is depicted as a region of relatively low signal intensity.

Figures 2 (A)-(D) illustrate changes in image intensity during transit of (A) 0.005, (B) 0.010, (C) 0.020, and (D) 0.040 mmol/kg MnDPDP in rats subjected to coronary artery occlusion (n = 7-8 per group). Values are expressed as a percentage of fully relaxed intensity. MnDPDP caused a sharp effect on LV chamber blood and myocardial signal. The myocardial enhancement profile shows no evidence of rapid clearance of the contrast agent.

Figure 2(E) illustrates the effect of various doses of MnDPDP on normal myocardium. Values are expressed as a percentage of fully relaxed intensity.

During and after the transit of MnDPDP the ischemic region was depicted as a cold-spot. Delineation of the ischemic region persisted for several minutes after the entry of MnDPDP. The ischemic region was not clearly visualized before the bolus passage, and was indistinguishable from LV chamber blood. At the lowest dose, 0.005 mmol/kg, there was clear signal enhancement in LV chamber blood. However, the slight enhancement of normally perfused region was insufficient to provide clear delineation of the ischemic region. At the higher doses (0.010, 0.020, and 0.040 mmol/kg), the ischemic region was identified as a region of very low signal, in contrast to hyperintense normal myocardium and LV chamber blood. The area and severity of ischemia was found to correlate closely with the corresponding phthalocyanine blue-stained histopathological sections.

Baseline intensity of the myocardium was 15 to 20% of the fully relaxed value. MnDPDP demonstrated a clear bolus profile at all doses of contrast agent, with peak myocardial enhancement preceded by peak enhancement of left ventricular chamber blood. Myocardial enhancement increased incrementally with dose over the entire range of MnDPDP (5, 10, 20, 40 micromole/kg) up to $23\pm2$, $28\pm2$, $34\pm5$, and $48\pm4$% of fully relaxed images, respectively.

<u>Study 2</u>

The aim of the study was to examine myocardial uptake and retention of manganese and corresponding changes in tissue $T_1$ during normal perfusion conditions and during early and late stages of subtotal ischemia. The study also aimed at assessments of cardiac function and energy metabolism and was undertaken in ex vivo guinea pig hearts.

MATERIALS AND METHODS:

Guinea pigs (450-500 g) were anaesthetised with sodium pentobarbital (intraperiotenal injection 100 mg). Hearts were rapidly excised and connected to the aortic cannula of a standard Langendorff perfusion system with conditions preset at 37°C. Perfusion with glucose-containing Krebs Henseleit's bicarbonate buffer (Krebs buffer) was maintained in the constant flow mode during the entire experiment by use of a finely adjustable pump. Under normal perfusion conditions coronary flow rate (CFR) was 30 ml/min and during global, low flow ischemia 0.25 ml/min (92% reduction of normal CFR). Left ventricular (LV) developed pressure (LVDP) was measured by use of a LV fluid-filled balloon connected to a pressure transducer and recording device. At the end of the experiments excised samples from the LV were freeze-clamped, freeze-dried and prepared for analysis of LV contents of Mn metal by atomic absorbtion spectrophotometry and of ATP by high pressure liquid chromatography. Fresh, non-clamped LV samples were taken for immediate (within 30 min) measurements of tissue longitudinal ($T_1$) relaxation time by use of a Bruker Minispec PC 110 spectrometer operating at 0.234 T and at a proton frequency of 10 MHz.

In normal perfusion experiments (series A) hearts were subjected to a 20 min control period before being perfused for 5 min with ascending concentrations (0-3000 $\mu$M) of MnDPDP present in the Krebs buffer. This was followed by 5 min perfusion with buffer only to ensure an effective washout of residual extracellular MnDPDP. In global ischemia experiments hearts were perfused at low flow (92% CFR reduction) with normal Krebs buffer. After different durations (0, 15, 30, 90, and 120 min) of ischemia MnDPDP (1000 $\mu$M, 3000 $\mu$M) was included in the buffer for a final 5 min period

of low flow ischemia. The experiments were ended by 5 min perfusion at normal flow with anoxic and calcium-free Krebs buffer to ensure washout of residual extracellular MnDPDP without reactivating normal cardiac function. In the results (series B) the ischemia durations are presented as 0-5 min, 15-20 min, 30-35 min, 90-95 min, and 120-125 min.

RESULTS:

A. Normal perfusion conditions

Figure 3 illustrates changes in LVDP during normal perfusion with Krebs buffer in the presence (10-15 min) and absence (15-20 min) of MnDPDP. Values are expressed as a percentage of values obtained during control perfusion. n = 6. Mean values are presented.

Figure 3 shows that LVDP falls rapidly during perfusion with higher concentrations of MnDPDP and that LVDP returns rapidly to control values during the following 5 min of MnDPDP washout. These findings are in accordance with the divalent Mn ion ($Mn^{2+}$) functioning as a slow calcium channel blocker.

Table 1 below shows how increasing concentrations of MnDPDP in the buffer in a stepwise manner raise the LV content of Mn and how tissue $T_1$ values fall correspondingly. In control hearts (not perfused with MnDPDP) the mean values were 4.1 μmole/100 g dry wt for Mn content and 1046 msec for $T_1$. MnDPDP 300 μM, a concentration with only a marginal 10% depression of LVDP, caused a 5-6 fold increase in Mn content and a 40% reduction in $T_1$. Perfusion with 1000 μM and 3000 μM of MnDPDP led to an 11-fold and a 17-fold rise in Mn content respectively and to a 55% and a 70% reduction in $T_1$ values. Since the experiments ended with a thorough 5 min washout of extracellular MnDPDP, this indicates that the efficacy of cardiac tissue in cellular uptake and retention of Mn is high under normal perfusion conditions.

Table 1 - Tissue Mn content (μmole/100 g dry wt) and $T_1$ relaxation time (msec) following exposure to MnDPDP (0-3000 μM) during normal perfusion conditions. n=6. Mean values and standard deviation (SD) are shown.

| MnDPDP | 0μM | 30μM | 100μM | 300μM | 1000μM | 3000μM |
|---|---|---|---|---|---|---|
| Mn-content | 4.1(1.0) | 11.0(1.5) | 12.2(2.4) | 23.2(4.1) | 45.4(10.8) | 70.4(14.9) |
| $T_1$ | 1046(164) | 871(93) | 808(81) | 619(18) | 461(85) | 341(46) |

B. Low flow ischemia

Table 2 below presents tissue ATP values and their fall during ischemia of increasing duration. Particularly it can be seen that the fall is gradual and only moderate during the initial phase. Thus at 15-20 min and 30-35 min ATP values are respectively 22% and 31% lower than the 0-5 min values, whereas after 90-95 min ATP values are reduced by 88%. Hearts reperfused for 45 min after 15-20 min and 90-95 min of low flow ischemia showed widely different recoveries of LVDP, 62% and 12% respectively. Taken together with the ATP values this indicates that 15-20 min of ischemia was associated with a gradually reversible ischemic injury, whereas hearts were irreversibly injured after 90-95 min of ischemia.

Table 2 - Tissue ATP content (μmole/g dry wt) and postischemic recovery of LVDP(%) after increasing duration of low flow ischemia (min). n=4. Hearts were freeze-clamped at end of ischemia for ATP analysis. Hearts were reperfused after 15-20 min and 90-95 min of ischemia and LVDP in % of preischemic control values is presented after 45 min of reperfusion. Mean values and standard deviation (SD) are shown.

| Ischemia | 0-5 min | 15-20 min | 30-35 min | 90-95 min |
|---|---|---|---|---|
| ATP content | 25.3(7.1) | 19.8(3.9) | 17.4(4.2) | 3.0(1.5) |
| LVDP recovery(%) | --------- | 62(6) | --------- | 12(3) |

Table 3 below shows that MnDPDP 1000 μM and 3000 μM applied at onset (0-5 min) of ischemia raised tissue Mn content above the control level in non-treated hearts (Table 1) by a factor of respectively 2-3 and 4-5. A tendency to falling Mn values was seen already after 15-20 min ischemia. After ischemic durations of 30-35 min, 90-95 min, and 120-125 min tissue Mn content was reduced considerably, and the mean values were not significantly different from those found in normally perfused non-treated hearts (Table 1). These data show that ischemia alters the mechanisms for manganese uptake thus blocking cellular uptake and accumulation of Mn at an early stage. Also, when comparing tissue Mn contents from early ischemic conditions (Table 3, 0-5 min) with those from normal perfusion conditions (Table 1), it can be seen that the latter contents are approximately 4 times higher. This means that two nonseparable factors

reduce tissue Mn content immediately or shortly after onset of ischemia: reduction in flow (and pressure) from the normally perfused state to the ischemic state inducing a state of acute hibernation; and the rapid onset of metabolic alterations during ischemia.

Table 3 - Tissue Mn content (μmole/100 g dry wt) after increasing duration of low flow ischemia (min) with administration of MnDPDP 1000 μM or 3000 μM during the last 5 min. n=4. Mean values and standard deviation (SD) are shown.

| Ischemia | 0-5 min | 15-20 min | 30-35 min | 90-95 min | 120-125 min |
|---|---|---|---|---|---|
| MnDPDP 1000 μM | 10.5(3.6) | 9.9(2.3) | 4.3(1.6) | 5.7(2.4) | 5.6(2.3) |
| MnDPDP 3000 μM | 16.5(2.4) | 12.9(4.0) | 6.0(2.2) | 7.2(2.8) | ------- |

The accumulated data from normal flow conditions (series A experiments) show that myocardial uptake and accumulation of Mn with MnDPDP is a rapid process in the normal and well-perfused heart. A rise in the Mn content of cardiac cells is parallelled by a reduction in tissue $T_1$ longitudinal relaxation time and forms the basis for increase in signal intensity with MnDPDP in MRI.

The accumulated data from ischemia experiments (series B) show that the cardiac cell uptake of Mn from MnDPDP is reduced or abolished during early ischemia, i.e. well before the onset of irreversible tissue injury.

The accumulated data from both series of experiments indicate that MnDPDP may function as a potentially effective MRI marker of normal cardiac function.

<u>Study 3</u>

The aim of the study was to examine the effect of dose on ΔR1 change in normal and reperfused infarcted myocardium following intravenous MnDPDP administration.

MATERIALS AND METHODS:

Sprague-Dawley rats (female, 270-320g, n=24) were subjected to occlusion of the anterior branch of the left main coronary artery for 60 minutes, followed by reperfusion for 120 minutes before injecting the test agent. A catheter was placed in the tail vein for administration of contrast media. Animals were connected to an ECG patient monitor and placed in the magnet such that the heart of each rat was at or near ioscenter. Magnetic field was optimized for a central slab of 6 mm thickness, within which the 2 mm section to be imaged was located. Inversion and slice select pulses were calibrated. Inversion recovery echo planar imaging was then used to measure baseline T1 values for myocardium and left ventricle chamber blood.

MnDPDP was then administered at doses of 25, 50 and 100 μmole/kg by intravenous injection of 2.5, 5 and 10 ml/kg, respectively, of the test solution which contained a 10 mM solution of MnDPDP. These injections were followed by a flush of physiologic saline solution (0.9%) such that the combined volumes of test agent and saline flush was 11 ml/kg. All injected solutions were warmed before being administered and were injected slowly over 1.5 mins. T1 values were remeasured at 5 minute intervals during the first 60 minutes after injection. Occasional animals were imaged at 90 minutes for T1 determination (EPI) and for visual inspection of contrast on STIR (short TI inversion recovery) prepared spin echo images.

After imaging was completed, animals were sacrificed by an overdose of pentobarbital and the heart was excised, sectioned and stained in triphenyltetrazolium chloride solution to define the infarcted region.

RESULTS:

Each heart contained an infarcted region in the anterior and lateral walls of the left ventricle. The infarcted region was located in a similar position to a region which behaved abnormally on contrast enhanced imaging. The abnormal region showed different T1 values from normal myocardium (septum and posterior wall) before and after contrast administration. For the purposes of this study, the abnormal region noted on MRI is hereinafter considered to represent the infarcted region.

$T_1$ values of myocardium (normal and infarcted) and chamber blood were measurably reduced following all doses of the test agent. The magnitude of T1 change increased with dose for all regions (see Table 4 below). The test agent cleared from both blood and infarcted myocardium but accumulated in uninfarcted myocardium. Over the course of 60 minutes after injection, the change in R1 (ΔR1 = 1/T1post - 1/T1pre) of infarcted myocardium and chamber blood

diminished with time but that of normal myocardium increased with time (Figure 4).

Clearance of ΔR1 from blood and infarcted myocardium (Figures 4a, 4b and Table 5 below) was well fit by a biexponential function (eqn 1), but not a monoexponential function.

$$\Delta R1 = K1 \exp(-E1t) + K2 \exp(-E2t) \qquad [1]$$

where K's and E's are constants and t is time (in min). The increase in ΔR1 in normal myocardium was well fit by a linear function for each dose (Figure 4c and Table 5 below). The apparent clearance or accumulation rates did not show an appreciable dose dependence.

Plots of ΔR1 ratio (tissue/blood) showed a slight decline with time for infarcted myocardium, consistent with the view that test agent, as well as any uncomplexed manganese, is as efficiently cleared from infarcted myocardium as from the blood pool. On the other hand, plots of ΔR1 ratio increased with time for normal myocardium (Figure 5). The curves were well fit by linear functions with slopes showing an inverse relation to administered dose. It is notable that even after one hour post injection with very low quantities of MnDPDP in the blood pool, there is no apparent slowing in the build-up of manganese in the normal myocardium after the lowest dose. This suggests that considerable contrast enhancement may be obtained several hours after administration, even at quite low dose of MnDPDP, particularly given the rather slow E2 component of ~0.01 per min (equivalent to -70 minutes plasma half life).

In cases imaged at 90 minutes post injection (2 or 3 at each dose), conventional T1 weighted images showed little contrast (SI normal/SI infarct = 1.15) between normal and infarcted myocardium among animals which received the highest dose and about 1.25 for the lowest dose (n=1). On the other hand, STIR sequences (TI= 200-400 ms, TR = 600 ms) showed very good contrast (SI normal/SI infarct = 0.3 to 4.0, depending on TI). No contrast (SI normal/ SI infarct -1.06) was observed on STIR sequences (using these parameters) before contrast administration (n=1). Less contrast was observed on STIR sequences as the dose was decreased. At the lowest dose, contrast between infarcted and normal myocardium was subtle (SI normal/ SI infarct -0.85 - 1.3 and low signal to noise, somewhat higher values were obtained with TR increased to -1.0 sec).

From an examination of the difference in R1 values between normal and reperfused infarcted myocardium produced by the doses of agent given (Figure 6), it is clear that there is not much difference among the groups. This suggests that, in principle, it should be possible to achieve approximately the same contrast for all dose groups. However, as the dose is decreased, TR must be increased.

It is clear from the data that if R1 continues to rise in normal myocardium, while returning to pre-contrast values for the infarcted region, the maximal contrast will be a function of the R1 difference between infarct and normal at the peak of ΔR1 in normal myocardium. This point should come at somewhat before 4 hours after contrast administration, given the rather slow clearance kinetics from the blood pool.

Table 4 - T1 values measured after injection of MnDPDP

| Time | Dose (μmol/kg) | Blood | T1 (sec) normal | Infarct |
|---|---|---|---|---|
| pre | | 1.31±0.02 | 0.89±0.02 | 1.16±0.02 |
| 5 | | 0.63±0.03 | 0.59±0.01 | 0.46±0.02 |
| 10 | | 0.74±0.03 | 0.59±0.01 | 0.47±0.02 |
| 15 | | 0.80±0.02 | 0.58±0.01 | 0.51±0.02 |
| 20 | | 0.83±0.02 | 0.57±0.01 | 0.55±0.03 |
| 25 | | 0.87±0.02 | 0.57±0.01 | 0.58±0.03 |
| 30 | 100 | 0.89±0.02 | 0.55±0.01 | 0.60±0.03 |
| 35 | | 0.91±0.03 | 0.54±0.01 | 0.61±0.03 |
| 40 | | 0.92±0.03 | 0.54±0.01 | 0.63±0.03 |
| 45 | | 0.93±0.03 | 0.53±0.01 | 0.65±0.03 |
| 50 | | 0.94±0.03 | 0.53±0.01 | 0.66±0.03 |
| 55 | | 0.97±0.02 | 0.52±0.01 | 0.69±0.03 |
| 60 | | 0.97±0.03 | 0.52±0.01 | 0.69±0.03 |
| pre | | 1.32±0.01 | 0.89±0.01 | 1.19±0.01 |
| 5 | | 0.84±0.02 | 0.69±0.01 | 0.59±0.01 |
| 10 | | 0.93±0.03 | 0.68±0.01 | 0.65±0.01 |
| 15 | | 0.97±0.02 | 0.67±0.01 | 0.69±0.01 |

(continued)

| Time | Dose (μmol/kg) | Blood | T1 (sec) normal | Infarct |
|---|---|---|---|---|
| 20 | | 1.00±0.02 | 0.67±0.01 | 0.73±0.01 |
| 25 | | 1.02±0.03 | 0.64±0.01 | 0.76±0.02 |
| 30 | 50 | 1.04±0.03 | 0.63±0.01 | 0.80±0.01 |
| 35 | | 1.05±0.03 | 0.62±0.01 | 0.82±0.02 |
| 40 | | 1.06±0.03 | 0.60±0.01 | 0.85±0.03 |
| 45 | | 1.08±0.03 | 0.60±0.01 | 0.87±0.02 |
| 50 | | 1.08±0.03 | 0.59±0.01 | 0.88±0.02 |
| 55 | | 1.08±0.03 | 0.58±0.01 | 0.89±0.03 |
| 60 | | 1.10±0.03 | 0.58±0.01 | 0.91±0.03 |
| pre | | 1.36±0.01 | 0.91±0.01 | 1.21±0.01 |
| 5 | | 1.04±0.02 | 0.77±0.01 | 0.76±0.02 |
| 10 | | 1.11±0.02 | 0.76±0.01 | 0.82±0.02 |
| 15 | | 1.14±0.02 | 0.75±0.01 | 0.86±0.03 |
| 20 | | 1.16±0.02 | 0.73±0.01 | 0.90±0.03 |
| 25 | | 1.18±0.02 | 0.72±0.01 | 0.92±0.03 |
| 30 | 25 | 1.18±0.02 | 0.70±0.01 | 0.93±0.03 |
| 35 | | 1.19±0.02 | 0.70±0.01 | 0.95±0.03 |
| 40 | | 1.20±0.02 | 0.69±0.01 | 0.98±0.03 |
| 45 | | 1.21±0.02 | 0.68±0.01 | 0.99±0.03 |
| 50 | | 1.22±0.02 | 0.67±0.01 | 1.00±0.03 |
| 55 | | 1.22+0.02 | 0.67±0.01 | 1.01±0.03 |
| 60 | | 1.23±0.02 | 0.65±0.01 | 1.02±0.03 |

Table 5 - Clearance data

| Bi-exponential fit, clearance from blood and infarct | | | | | |
|---|---|---|---|---|---|
| dose (μm) | ROI | K1 | E1 ($min^{-1}$) | K2 | E2 ($min^{-1}$) |
| 25 | blood | 0.25±0.03 | 0.21±0.03 | 0.16±0.01 | 0.011±0.001 |
| 25 | infarct | 0.30±0.03 | 0.15±0.03 | 0.38±0.02 | 0.015±0.001 |
| 50 | blood | 0.38±0.02 | 0.15±0.03 | 0.28±0.01 | 0.009±0.001 |
| 50 | infarct | 0.70±0.08 | 0.058±0.03 | 0.34±0.09 | 0.005±0.003 |
| 100 | blood | 0.85±0.05 | 0.18±0.02 | 0.51±0.02 | 0.011±0.001 |
| 100 | infarct | 0.61±0.13 | 0.11+0.06 | 1.11+0.19 | 0.010±0.003 |
| linear fit - increase in normal myocardium | | | | | |
| dose | | ROI | intercept | slope ($min^{-1}$) | R |
| 25 | | normal | 0.187 | 0.0043 | 0.995 |
| 50 | | normal | 0.294 | 0.0056 | 0.989 |
| 100 | | normal | 0.523 | 0.0050 | 0.991 |

**Claims**

1. Use of a physiologically acceptable manganese complex or salt thereof for the manufacture of a contrast medium for use in a method of detecting myocardial ischemia in a human or non-human body, said method comprising administering said complex or salt thereof to said body at a dosage of 0.001 to 0.2 mmol/kg bodyweight, subjecting said body to an inversion recovery echo planar imaging procedure capable of generating images with time intervals of less than 0.5 seconds and thereafter providing a series of images of the myocardium of said body whereby to identify regions of abnormal blood flow.

2. Use as claimed claim 1 wherein said manganese complex is a manganese chelate complex having a $K_a$ value of from $10^7$ to $10^{25}$.

3. Use as claimed in claim 2 wherein said manganese chelate comprises a chelating compound of formula I :

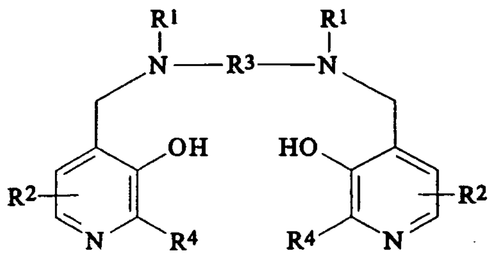

(I)

or a salt thereof
(wherein in formula I

each $R^1$ independently represents hydrogen or $-CH_2COR^5$ ;
$R^5$ represents hydroxy, optionally hydroxylated alkoxy, amino or alkylamido;
each $R^2$ independently represents a group $XYR^6$;
X represents a bond, or a $C_{1-3}$ alkylene or oxoalkylene group optionally substituted by a group $R^7$;
Y represents a bond, an oxygen atom or a group $NR^6$;
$R^6$ is a hydrogen atom, a group $COOR^8$, an alkyl, alkenyl, cycloalkyl, aryl or aralkyl group optionally substituted by one or more groups selected from $COOR^8$, $CONR^8_2$, $NR^8_2$, $OR^8$, $=NR^8$, =O, $OP(O)(OR^8)R^7$ and $OSO_3M$;
$R^7$ is hydroxy, an optionally hydroxylated, optionally alkoxylated alkyl or aminoalkyl group;
$R^8$ is a hydrogen atom or an optionally hydroxylated, optionally alkoxylated alkyl group;
M is a hydrogen atom or one equivalent of a physiologically tolerable cation;
$R^3$ represents a $C_{1-8}$ alkylene group, a 1,2-cycloalkylene group, or a 1,2-arylene group; and
each $R^4$ independently represents hydrogen or $C_{1-3}$ alkyl).

4. Use as claimed in claim 3 wherein in formula I:

$R^5$ is hydroxy, $C_{1-8}$ alkoxy, ethylene glycol, glycerol, amino or $C_{1-8}$ alkylamido;
X is a bond or a group selected from $CH_2$, $(CH_2)_2$, CO, $CH_2CO$, $CH_2CH_2CO$ or $CH_2COCH_2$;
Y is a bond;
$R^6$ is a mono- or poly(hydroxy or alkoxylated) alkyl group or a group of the formula OP (O) $(OR^8)R^7$; and
$R^7$ is hydroxy or an unsubstituted alkyl or aminoalkyl group.

5. Use as claimed in claim 3 or claim 4 wherein in formula I, $R^3$ is ethylene and each group $R^1$ represents $-CH_2COR^5$ in which $R^5$ is hydroxy.

6. Use as claimed in any one of claims 3 to 5 in which the compound of formula I is N,N'-bis-(pyridoxal-5-phosphate)-ethylenediamine-N,N'-diacetic acid (DPDP) or N,N'-dipyridoxyl-ethylenediamine-N,N'-diacetic acid (PLED) .

7. Use as claimed in any of the preceding claims wherein said magnetic resonance imaging procedure is carried out within a period of up to 6 hours after the administration of said complex or salt thereof to said body.

8. Use as claimed in any of the preceding claims wherein the contrast medium further comprises calcium chelate complexes.

9. Use as claimed in any of the preceding claims wherein the contrast medium further comprises calcium or sodium salts.

10. Use as claimed in claim 9 wherein the calcium salt comprises calcium chloride, calcium ascorbate, calcium gluconate

or calcium lactate.

**11.** Use as claimed in any of the preceding claims wherein the contrast medium further comprises physiologically compatible buffers.

**12.** Use as claimed in any of the preceding claims wherein the contrast medium further comprises an antioxidant such as ascorbic acid or a reducing sugar.

**13.** Use as claimed in claim 2 wherein said chelate complex is a complex of a linear, branched or macrocyclic chelant selected from polyaminopolycarboxylic acid chelants and carboxylic acid derivatives thereof.

**14.** Use as claimed in any preceding claim wherein said magnetic resonance imaging procedure is one capable of generating images with time intervals of less than 100 milliseconds.

**15.** Use as claimed in claim 1 wherein said imaging procedure is one in which TI (inversion time) is 100 to 800 msecs, TR (repetition time) is 2000 msecs and TE (echo time) is less than 20 msecs.

**16.** Use as claimed in any preceding claim wherein said manganese complex or salt thereof is administered at a dosage of 0.005 to 0.2 mmol/kg bodyweight.

**17.** Use as claimed in any preceding claim wherein said manganese complex or salt thereof is administered at a dosage of 0.01 to 0.05 mmol/kg bodyweight.

**18.** Use of a physiologically acceptable manganese complex or salt thereof for the manufacture of a contrast medium for use in a method of evaluating the severity of myocardial ischemia in a human or non-human body, said method comprising administering said complex or salt thereof to said body at a dosage of 0.001 to 0.2 mmol/kg bodyweight, subjecting said body to an inversion recovery echo planar imaging procedure capable of generating images with time intervals of less than 0.5 seconds and thereafter providing a series of images of the myocardium of said body whereby to indicate the degree of blood perfusion deficit in the myocardium.

**19.** Use of a physiologically acceptable manganese complex or salt thereof for the manufacture of a contrast medium for use in a method of monitoring reperfusion of the myocardium of a human or non-human body, said method comprising administering said complex or salt thereof to said body at a dosage of 0.001 to 0.2 mmol/kg bodyweight, subjecting said body to an inversion recovery echo planar imaging procedure capable of generating images with time intervals of less than 0.5 seconds and thereafter providing a series of images of the myocardium of said body whereby to identify regions of reperfusion.

**20.** Use of a physiologically acceptable manganese complex or salt thereof for the manufacture of a contrast medium for use in a method of discriminating between reversibly and irreversibly injured myocardial tissue in a human or non-human body, said method comprising administering said complex or salt thereof to said body at a dosage of 0.001 to 0.2 mmol/kg bodyweight, subjecting said body to an inversion recovery echo planar imaging procedure capable of generating images with time intervals of less than 0.5 seconds and thereafter providing a series of images of the myocardium of said body whereby to discriminate reversibly from irreversibly injured tissue.

**21.** Use of a physiologically acceptable manganese complex or salt thereof for the manufacture of a contrast medium for use in a method of distinguishing viable myocardial tissue from necrotic (infarcted) tissue in a human or non-human body, said method comprising administering said complex or salt thereof to said body at a dosage of 0.001 to 0.2 mmol/kg bodyweight, subjecting said body to an inversion recovery echo planar imaging procedure capable of generating images with time intervals of less than 0.5 seconds and thereafter providing a series of images of the myocardium of said body whereby to distinguish viable myocardial tissue from infarcted tissue.

**22.** A method of imaging the myocardium in a human or non-human body previously administered with a physiologically acceptable manganese complex or salt thereof at a dosage of 0.001 to 0.2 mmol/kg bodyweight, said method comprising subjecting said body to an inversion recovery echo planar imaging procedure capable of generating images with time intervals of less than 0.5 seconds and thereafter providing a series of images of the myocardium of said body.

## Patentansprüche

1. Verwendung eines physiologisch annehmbaren Mangan-Komplexes oder Salzes davon zur Herstellung eines Kontrastmediums zur Verwendung in einem Verfahren zum Detektieren einer myokardialen Ischämie in einem menschlichen oder nicht-menschlichen Körper, wobei das Verfahren umfasst das Verabreichen des Komplexes oder des Salzes davon an den Körper bei einer Dosierung von 0,001 bis 0,2 mmol/kg Körpergewicht, Unterziehen des Körpers einer Inversion-Recovery-Echo-Planar-Bildgebungsprozedur, die fähig ist, Bilder mit Zeitintervallen von weniger als 0,5 Sekunden zu erzeugen, und danach Liefern eine Reihe von Bildern des Myokards des Körpers, um **dadurch** Bereiche abnormalen Blutflusses zu identifizieren.

2. Verwendung nach Anspruch 1, wobei der Mangan-Komplex, ein Mangan-Chelatkomplex mit einem $K_a$-Wert von $10^7$ bis $10^{25}$ ist.

3. Verwendung nach Anspruch 2, wobei das Mangan-Chelat eine chelatierende Verbindung der Formel I umfasst:

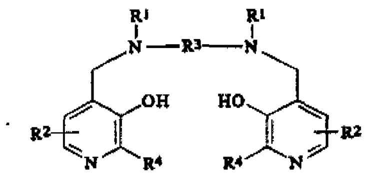

(I)

oder ein Salz davon
(wobei in Formel I

jedes $R^1$ unabhängig Wasserstoff oder $-CH_2COR^5$ repräsentiert;
$R^5$ Hydroxy, ggf. hydroxyliertes Alkoxy, Amino oder Alkylamido repräsentiert;
jedes $R^2$ unabhängig eine Gruppe $XYR^6$ repräsentiert;
X eine Bindung oder eine $C_{1-3}$-Alkylen- oder Oxoalkylen-Gruppe, ggf. substituiert mit einer Gruppe $R^7$, repräsentiert;
Y eine Bindung, ein Sauerstoffatom oder eine Gruppe $NR^6$ repräsentiert;
$R^6$ steht für ein Wasserstoffatom, eine Gruppe $COOR^8$, eine Alkyl-, Alkenyl-, Cycloalkyl-, Aryl- oder Aralkyl-Gruppe, ggf, substituiert mit einer oder mehreren Gruppen, ausgewählt aus $COOR^8$, $CONR^8_2$, $NR^8_2$, $OR^8$, $=NR^8$, =0, OP (O) $(OR^8)$ $R^7$ und $OSO_3M$;
$R^7$ steht für Hydroxy, eine ggf. hydroxylierte, ggf. alkoxylierte Alkyl- oder Aminoalkyl-Gruppe;
$R^8$ steht für ein Wasserstoffatom oder eine ggf. hydroxylierte, ggf. alkoxylierte Alkylgruppe;
M steht für ein Wasserstoffatom oder ein Äquivalent eines physiologisch tolerierbaren Kations;
$R^3$ repräsentiert eine $C_{1-8}$-Alkylen-Gruppe, eine 1,2-Cycloalkylen-Gruppe, oder eine 1,2-Arylen-Gruppe; und jedes $R^4$ repräsentiert unabhängig Wasserstoff oder $C_{1-3}$-Alkyl).

4. Verwendung nach Anspruch 3, wobei in Formel I:

$R^5$ steht für Hydroxy, $C_{1-8}$-Alkoxy, Ethylenglycol, Glyzerin, Amino oder $C_{1-8}$-Alkylamido;
X steht für eine Bindung oder eine Gruppe ausgewählt aus $CH_2$, $(CH_2)_2$, CO, $CH_2CO$, $CH_2CH_2CO$ oder $CH_2COCH_2$;
Y für eine Bindung steht;
$R^6$ steht für eine Mono- oder Poly(hydroxy-oder alkoxylierte)-Alkyl-Gruppe oder Gruppe der Formel OP(O) $(OR^8)$ $R^7$; und
$R^7$ steht für Hydroxy oder eine unsubstituierte Alkyl-oder Aminoalkyl-Gruppe.

5. Verwendung nach Anspruch 3 oder 4, wobei in Formel I $R^3$ steht für Ethylen und jede Gruppe $R^1$ repräsentiert $-CH_2COR^5$, in der $R^5$ für Hydroxy steht.

6. Verwendung nach einem der Ansprüche 3 bis 5, in der die Verbindung der Formel I steht für N,N'-Bis-(pyridoxal-5-phosphat)-ethylendiamin-N,N'-diessigsäure (DPDP) oder N,N'-Dipyridoxyl-ethylendiamin-N, N'-diessigsäure (PLED).

7. Verwendung nach einem der vorliegenden Ansprüche, wobei die Magnetresonanzbildgebungsprozedur ausgeführt wird innerhalb einer Zeitdauer von bis zu 6 Stunden nach der Verabreichung des Komplexes oder Salzes davon an den Körper.

8. Verwendung nach einem der vorstehenden Ansprüche, wobei das Kontrastmedium weiter umfasst Calciumchelatkomplexe.

9. Verwendung nach einem der vorstehenden Ansprüche, wobei das Kontrastmedium weiter umfasst Calcium- oder Natriumsalze.

10. Verwendung nach Anspruch 9, wobei das Calciumsalz umfasst Calciumchlorid, Calciumascorbat, Calciumgluconat oder Calciumlactat.

11. Verwendung nach einem der vorstehenden Ansprüche, wobei das Kontrastmedium weiter umfasst physiologisch kompatible Puffer.

12. Verwendung nach einem der vorstehenden Ansprüche, wobei das Kontrastmedium weiter umfasst ein Antioxidationsmittel, wie Ascorbinsäure oder einen reduzierenden Zucker.

13. Verwendung nach Anspruch 2, wobei der Chelatkomplex ein Komplex ist aus einem linearen, verzweigten oder makrocyclischen Chelatbildner, ausgewählt aus Polyamino-Polycarbonsäure-Chelatbildnern und Carbonsäure-Derivaten davon.

14. Verwendung nach einem der vorstehenden Ansprüche, wobei die Magnetresonanzbildgebungsprozedur eine ist, die fähig ist, Bilder mit Zeitintervallen von weniger als 100 Millisekunden zu erzeugen.

15. Verwendung nach Anspruch 1, wobei die Bildgebungsprozedur eine ist, in der TI (Inversionszeit) 100 bis 800 ms beträgt, TR (Wiederholungszeit) 2000 ms beträgt und TE (Echozeit) weniger als 20 ms beträgt.

16. Verwendung nach einem der vorstehenden Ansprüche, wobei der Mangan-Komplex oder das Salz davon verabreicht wird bei einer Dosierung von 0,005 bis 0,2 mmol/kg Körpergewicht.

17. Verwendung nach einem der vorstehenden Ansprüche, wobei der Mangan-Komplex oder das Salz davon verabreicht wird bei einer Dosierung von 0,01 bis 0,05 mmol/kg Körpergewicht.

18. Verwendung eines physiologisch annehmbaren Mangan-Komplexes oder Salzes davon zur Herstellung eines Kontrastmediums zur Verwendung in einem Verfahren zum Bewerten der Ernsthaftigkeit einer myokardialen Ischämie in einem menschlichen oder nicht-menschlichen Körper, wobei das Verfahren umfasst Verabreichen des Komplexes oder Salzes davon an den Körper bei einer Dosierung von 0,001 bis 0,2 mmol/kg Körpergewicht, Unterziehen des Körpers einer Inversion-Recovery-Echo-Planar-Bildgebungsprozedur, die fähig ist, Bilder mit Zeitintervallen von weniger als 0,5 Sekunden zu erzeugen und danach Liefern einer Reihe von Bildern des Myokards des Körpers, um **dadurch** den Grad des Blutperfusionsdefizits in Myokard anzuzeigen.

19. Verwendung eines physiologisch annehmbaren Mangankomplexes oder Salzes davon zur Herstellung eines Kontrastmediums zur Verwendung in einem Verfahren zum Überwachen der Reperfusion des Myokards eines menschlichen oder nicht-menschlichen Körpers, wobei das Verfahren umfasst Verabreichen des Komplexes oder Salzes davon an den Körper bei einer Dosierung von 0.001 bis 0,2 mmol/kg Körpergewicht, Unterziehen des Körpers einer Inversion-Recovery-Echo-Planar-Bildgebungsprozedur, die fähig ist, Bilder mit Zeitintervallen von weniger als 0,5 Sekunden zu erzeugen, und danach Liefern einer Reihe von Bildern des Myokards des Körpers, um **dadurch** Bereiche der Reperfusion zu identifizieren.

20. Verwendung eines physiologisch annehmbaren Mangankomplexes oder Salzes davon zur Herstellung eines Kontrastmediums zur Verwendung in einem Verfahren zum Unterscheiden zwischen reversibel und irreversibel verletzten Myokardgewebe in einem menschlichen oder nicht-menschlichen Körper, wobei das Verfahren umfasst Verabreichen des Komplexes oder Salzes davon an den Körper bei einer Dosierung von 0,001 bis 0,2 mmol/kg Körpergewicht, Unterziehen des Körpers einer Inversion-Recovery-Echo-Planar-Bildgebungsprozedur, die fähig ist, Bilder mit Zeitintervallen von weniger als 0,5 Sekunden zu erzeugen, und danach Liefern einer Reihe von Bildern des Myokards des Körpers, um **dadurch** zu unterscheiden zwischen reversibel und irreversibel verletztem Gewebe.

**21.** Verwendung eines physiologisch annehmbaren Mangankomplexes oder Salzes davon zur Herstellung eines Kontrastmediums zur Verwendung in einem Verfahren zum Unterscheiden eines lebensfähigen Myokardgewebes von nekrotischem (mit Infarkt behaftetem) Gewebe in einem menschlichen oder nicht-menschlichen Körper, wobei das Verfahren umfasst Verabreichen des Komplexes oder Salzes davon an den Körper bei einer Dosierung von 0,001 bis 0,2 mmol/kg Körpergewicht, Unterziehen des Körpers einer Inversion-Recovery-Echo-Planar-Bildgebungsprozedur, die fähig ist zum Erzeugen von Bildern mit Zeitintervallen von weniger als 0,5 Sekunden, und danach Liefern einer Reihe von Bildern des Myokards des Körpers, um **dadurch** lebensfiähiges Myokardgewebe von mit Infarkt behaftetem Gewebe zu unterscheiden.

**22.** Verfahren zur Bildgebung des Myokards in einem menschlichen oder nicht-menschlichen Körper, dem zuvor ein physiologisch annehmbarer Mangankomplex oder Salz davon bei einer Dosierung von 0,001 bis 0,2 mmol/kg Körpergewicht verabreicht wurde, wobei das Verfahren umfasst Unterziehen des Körpers einer Inversion-Recovery-Echo-Planar-Bildgebungsprozedur, die fähig ist zum Erzeugen von Bildern mit Zeitintervallen von weniger als 0,5 Sekunden, und danach Liefern einer Reihe von Bildern des Myokards des Körpers.

## Revendications

**1.** Utilisation d'un complexe de manganèse physiologiquement acceptable, ou un sel de ce complexe, pour la préparation d'un produit de contraste destiné à une méthode de détection d'une ischémie myocardique dans un corps humain ou non humain, ladite méthode consistant à administrer audit corps ledit complexe ou sel de ce complexe à raison de 0,001 à 0,2 mmol/kg de masse corporelle, à soumettre ledit corps à un procédé d'imagerie écho planaire en inversion-récupération capable de générer des images à des intervalles inférieurs à 0,5 seconde puis à produire une série d'images du myocarde dudit corps pour ainsi identifier des zones à écoulement sanguin anormal.

**2.** Utilisation selon la revendication 1, dans laquelle ledit complexe de manganèse est un complexe chélaté de manganèse ayant une valeur $K_a$ allant de $10^7$ à $10^{25}$.

**3.** Utilisation selon la revendication 2, dans laquelle le chélate de manganèse comprend un composé chélatant répondant à la formule I :

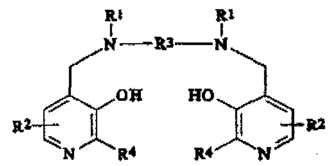

(I)

ou un sel de ce composé
(pour lequel, dans la formule I,

chaque $R^1$ représente indépendamment l'hydrogène ou $-CH_2COR^5$ ;
$R^5$ représente un groupe hydroxy, amino, alkylamido ou alcoxy éventuellement hydroxylé ;
chaque $R^2$ représente indépendamment un groupe $XYR^6$ ;
X représente une liaison, ou bien un groupe oxoalkylène ou alkylène en $C_1$-$C_3$ éventuellement substitué par un groupe $R^7$ ;
Y représente une liaison, un atome d'oxygène ou un groupe $NR^6$ ;
$R^6$ représente un atome d'hydrogène, un groupe $COOR^8$, un groupe alkyle, alcényle, cycloalkyle, aryle ou aralkyle éventuellement substitué par un ou plusieurs groupes sélectionnés parmi $COOR^8$, $CONR^8_2$, $NR^8_2$, $OR^8$, $=NR^8$, =0, $OP(O)(OR^8)R^7$ et $OSO_3M$ ;
$R^7$ représente hydroxy, un groupe alkyle ou aminoalkyle éventuellement hydroxylé ou alcoxylé ;

$R^8$ est un atome d'hydrogène ou un groupe alkyle éventuellement hydroxylé ou alaoxylé ;
M représente un atome d'hydrogène ou un équivalent d'un cation physiologiquement tolérable ;
$R^3$ représente un groupe alkylène en $C_1$-$C_8$, un groupe 1,2-cycloalkylène, ou un groupe 1,2-arylène ; et chaque $R^4$ représente indépendamment l'hydrogène ou un groupe alkyle en $C_1$-$C_3$).

**4.** Utilisation selon la revendication 3, dans laquelle, dans la formule I :

$R^5$ représente hydroxy, alcoxy en $C_1$-$C_8$, éthylène glycol, glycérol, amino ou alkylamido en $C_1$-$C_8$ ;
X représente une liaison ou un groupe sélectionné parmi $CH_2$, $(CH_2)_2$, CO, $CH_2CO$, $CH_2CH_2CO$ ou $CH_2COCH_2$ ;
Y représente une liaison ;
$R^6$ représente un groupe alkyle mono- ou poly(hydroxylé ou alcoxylé) ou un groupe répondant à la formule OP (O) $(OR^8)R^7$ ; et
$R^7$ représente hydroxy ou un groupe alkyle ou aminoalkyle non substitué.

**5.** Utilisation selon la revendication 3 ou la revendication 4, dans laquelle, dans la formule I, $R^3$ représente éthylène et chaque groupe $R^1$ représente $-CH_2COR^5$ où $R^5$ représente hydroxy.

**6.** Utilisation selon l'une quelconque des revendications 3 à 5, dans laquelle le composé de formule I est l'acide N,N'-bis,(pyridoxal-5-phosphate)-éthylènediamine-N,N'-diacétique (DPDP) ou l'acide N,N'-dipyridoxyl-ethylénediamine-N,N'-diacétique (PLED).

**7.** Utilisation selon l'une quelconque des revendications précédentes, dans laquelle le procédé d'imagerie par résonance magnétique est mis en oeuvre au plus tard dans les 6 heures suivant l'administration dudit complexe ou sel de celui-ci audit corps.

**8.** Utilisation selon l'une quelconque des revendications précédentes, dans laquelle le produit de contraste comprend en outre des complexes chélatés du calcium.

**9.** Utilisation selon l'une quelconque des revendications précédentes, dans laquelle le produit de contraste comprend en outre des sels de calcium ou de sodium.

**10.** Utilisation selon la revendication 9, dans laquelle le sel de calcium comprend le chlorure de calcium, l'ascorbate de calcium, le gluconate de calcium ou le lactate de calcium.

**11.** Utilisation selon l'une quelconque des revendications précédentes, dans laquelle le produit de contraste comprend en outre des tampons physiologiquement compatibles.

**12.** Utilisation selon l'une quelconque des revendications précédentes, dans laquelle le produit de contraste comprend en outre un antioxydant tel que l'acide ascorbique ou un sucre réducteur.

**13.** Utilisation selon la revendication 2, dans laquelle ledit complexe chélaté est un complexe de chélateur linéaire, ramifié ou macrocyclique sélectionné parmi les chélateurs à base d'acide polyaminopolycarboxylique et de dérivés d'acide carboxylique.

**14.** Utilisation selon l'une quelconque des revendications précédentes, dans laquelle ledit procédé d'imagerie par résonance magnétique est capable de générer des images à des intervalles inférieurs à 100 millisecondes.

**15.** Utilisation selon la revendication 1, dans laquelle ledit procédé d'imagerie est un procédé dans lequel TI (temps d'inversion) est compris dans la plage des 100 à 800 ms, TR (temps de répétition) est de 2 000 ms et TE (temps d'écho) est inférieur à 20 ms.

**16.** Utilisation selon l'une quelconque des revendications précédentes, dans laquelle ledit complexe de manganèse ou sel de celui-ci est administré à raison de 0,005 à 0,2 mmol/kg de masse corporelle.

**17.** Utilisation selon l'une quelconque des revendications précédentes, dans laquelle ledit complexe de manganèse ou sel de celui-ci est administré à raison de 0,01 à 0,05 mmol/kg de masse corporelle.

**18.** Utilisation d'un complexe de manganèse physiologiquement acceptable, ou d'un sel de ce complexe, pour la préparation d'un produit de contraste destiné à une méthode d'évaluation de la gravité d'une ischémie myocardique dans un corps humain ou non humain, ladite méthode consistant à administrer audit corps ledit complexe ou sel de ce complexe à raison de 0,001 à 0,2 mmol/kg de masse corporelle, à soumettre ledit corps à un procédé d'imagerie écho planaire en inversion-récupération capable de générer des images à des intervalles inférieurs à 0,5 seconde puis à produire une série d'images du myocarde dudit corps pour ainsi indiquer le degré de déficit d'irrigation sanguine du myocarde.

**19.** Utilisation d'un complexe de manganèse physiologiquement acceptable, ou d'un sel de ce complexe, pour la préparation d'un produit de contraste destiné à une méthode de surveillance de la reperfusion du myocarde d'un corps humain ou non humain, ladite méthode consistant à administrer audit corps ledit complexe ou sel de ce complexe à raison de 0,001 à 0,2 mmol/kg de masse corporelle, à soumettre ledit corps à un procédé d'imagerie écho planaire en inversion-récupération capable de générer des images à des intervalles inférieurs à 0,5 seconde puis à produire une série d'images du myocarde dudit corps pour ainsi identifier les zones à reperfusion.

**20.** Utilisation d'un complexe de manganèse physiologiquement acceptable, ou d'un sel de ce complexe, pour la préparation d'un produit de contraste destiné à une méthode d'établissement de distinction entre des tissus myocardiques présentant des lésions réversibles et ceux présentant des lésions irréversibles dans un corps humain ou non humain, ladite méthode consistant à administrer audit corps ledit complexe ou sel de ce complexe à raison de 0,001 à 0,2 mmol/kg de masse corporelle, à soumettre ledit corps à un procédé d'imagerie écho planaire en inversion-récupération capable de générer des images à des intervalles inférieurs à 0,5 seconde puis à produire une série d'images du myocarde dudit corps pour ainsi établir la distinction entre les tissus présentant des lésions réversibles et ceux présentant des lésions irréversibles.

**21.** Utilisation d'un complexe de manganèse physiologiquement acceptable, ou d'un sel de ce complexe, pour la préparation d'un produit de contraste destiné à une méthode d'établissement de distinction entre les tissus myocardiques viables et les tissus nécrotiques (infarcis) dans un corps humain ou non humain, ladite méthode consistant à administrer audit corps ledit complexe ou sel de ce complexe à raison de 0,001 à 0,2 mmol/kg de masse corporelle, à soumettre ledit corps à un procédé d'imagerie écho planaire en inversion-récupération capable de générer des images du myocarde à des intervalles inférieurs à 0,5 seconde puis à produire une série d'images du myocarde dudit corps pour ainsi établir une distinction entre les tissus myocardiques viables et les tissus infarcis.

**22.** Méthode d'imagerie du myocarde dans un corps humain ou non humain ayant précédemment reçu l'administration d'un complexe de manganèse physiologiquement acceptable, ou d'un sel de ce complexe, à raison de 0,001 à 0,2 mmol/kg de masse corporelle, ladite méthode consistant à soumettre ledit corps à un procédé d'imagerie écho planaire en inversion-récupération capable de générer des images à des intervalles inférieurs à 0,5 seconde puis à fournir une série d'images du myocarde dudit corps.

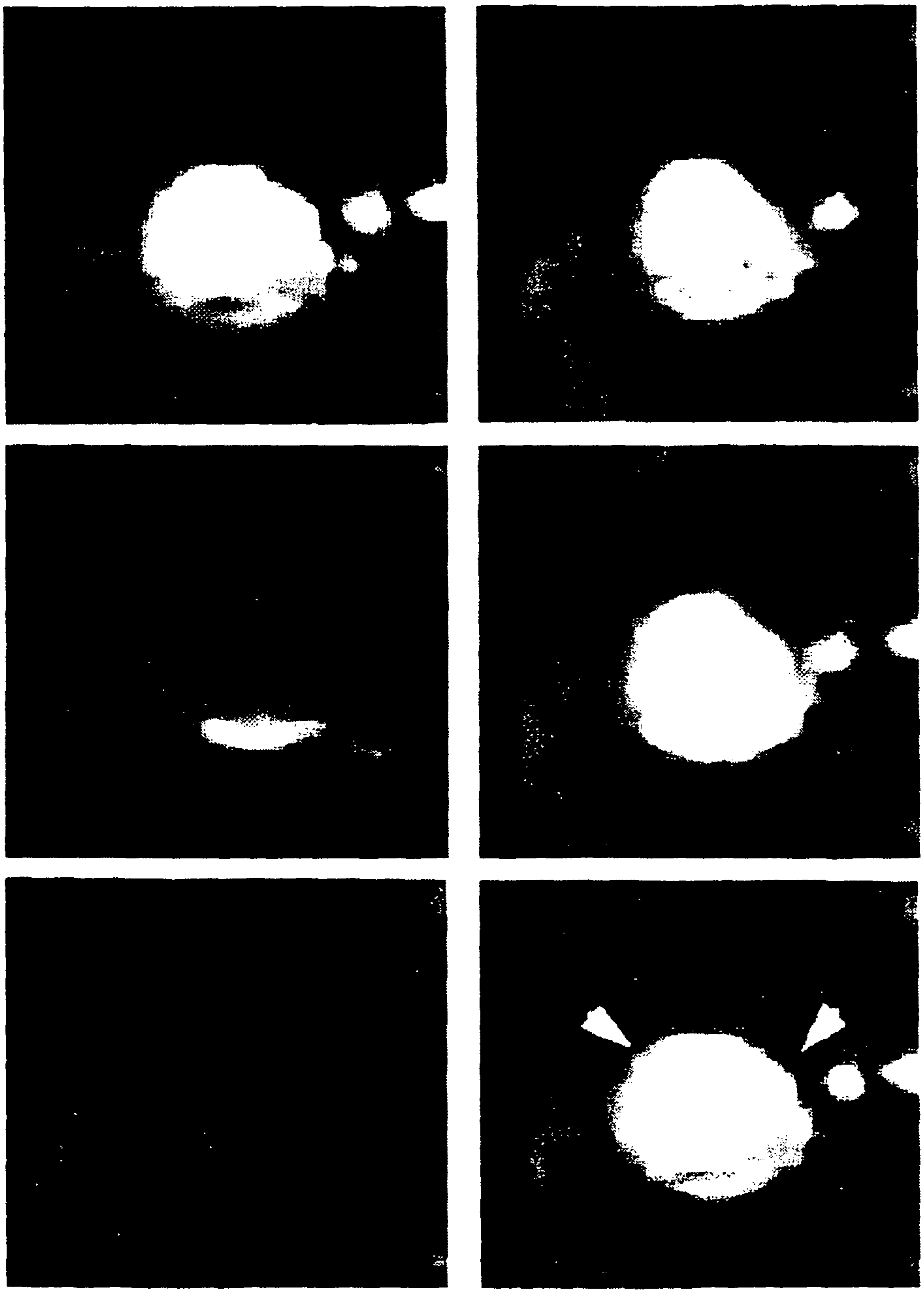

FIG. 1

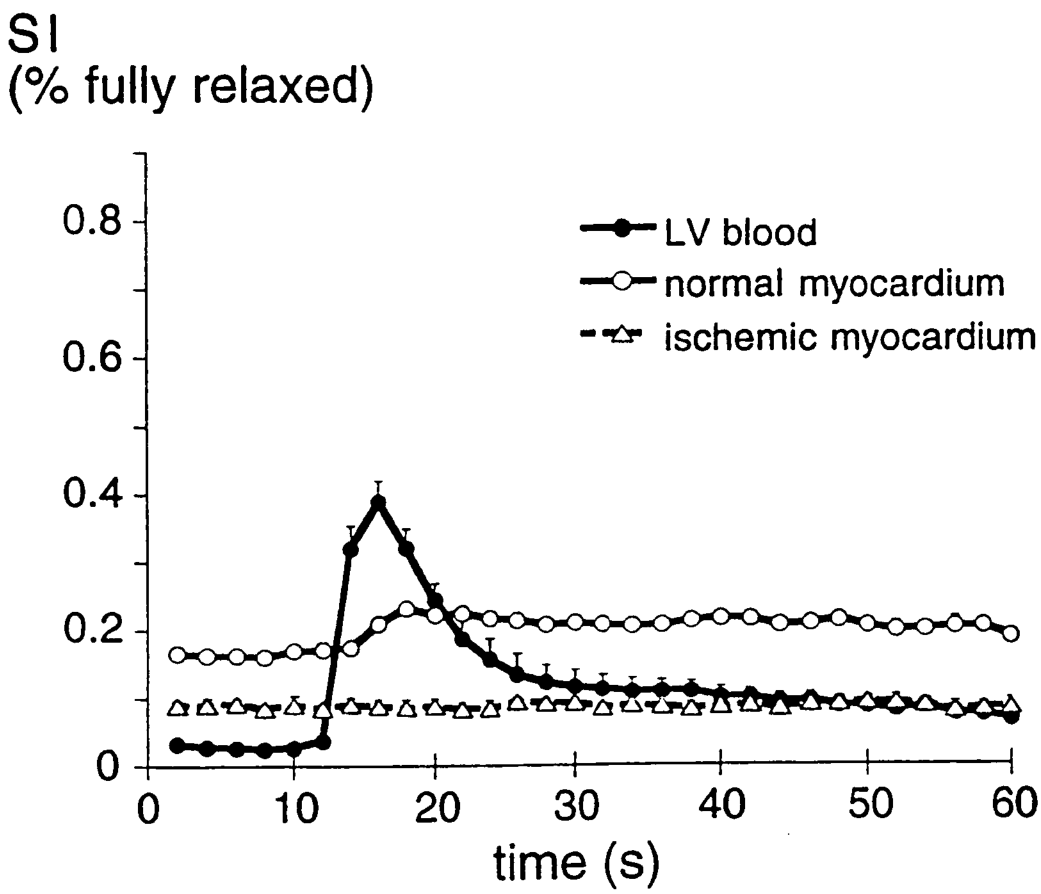
SI
(% fully relaxed)
0.8
0.6
0.4
0.2
0
0
10
20
30
40
50
60
time (s)
LV blood
normal myocardium
ischemic myocardium

FIG. 2A

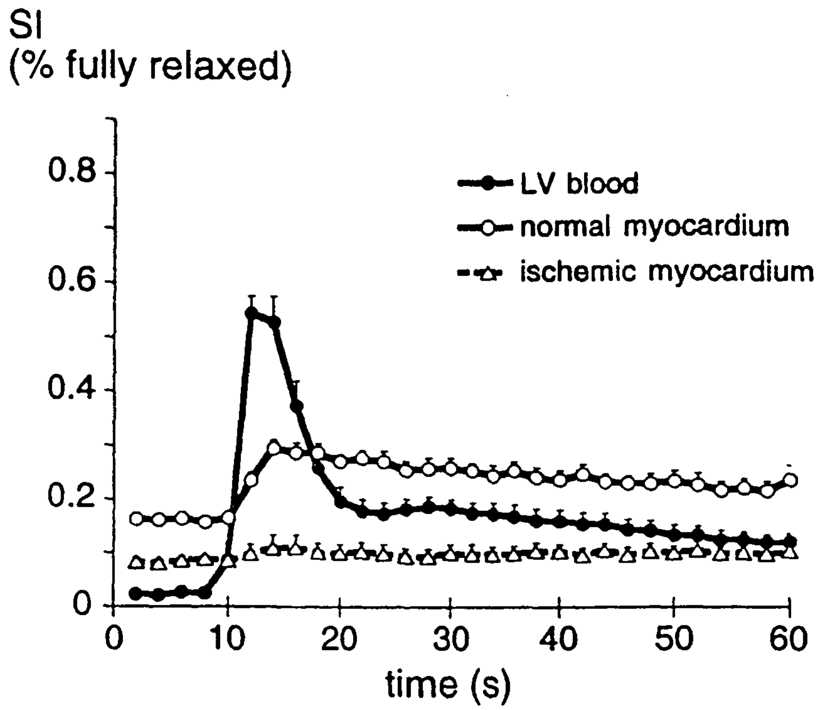
SI
(% fully relaxed)
0.8
0.6
0.4
0.2
0
0
10
20
30
40
50
60
time (s)
LV blood
normal myocardium
ischemic myocardium

FIG. 2B

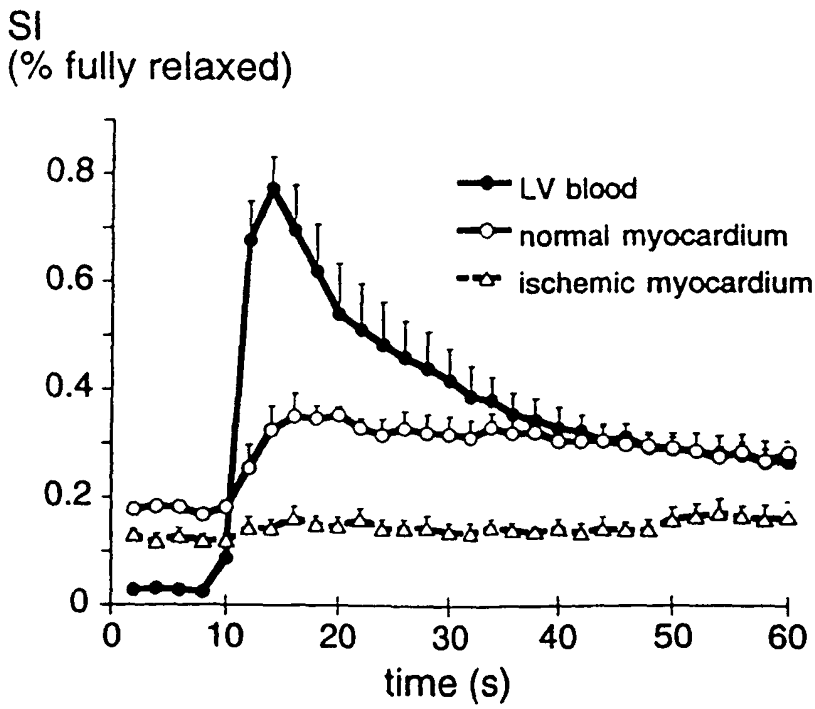
SI
(% fully relaxed)
0.8
0.6
0.4
0.2
0
0
10
20
30
40
50
60
time (s)
LV blood
normal myocardium
ischemic myocardium

FIG. 2C

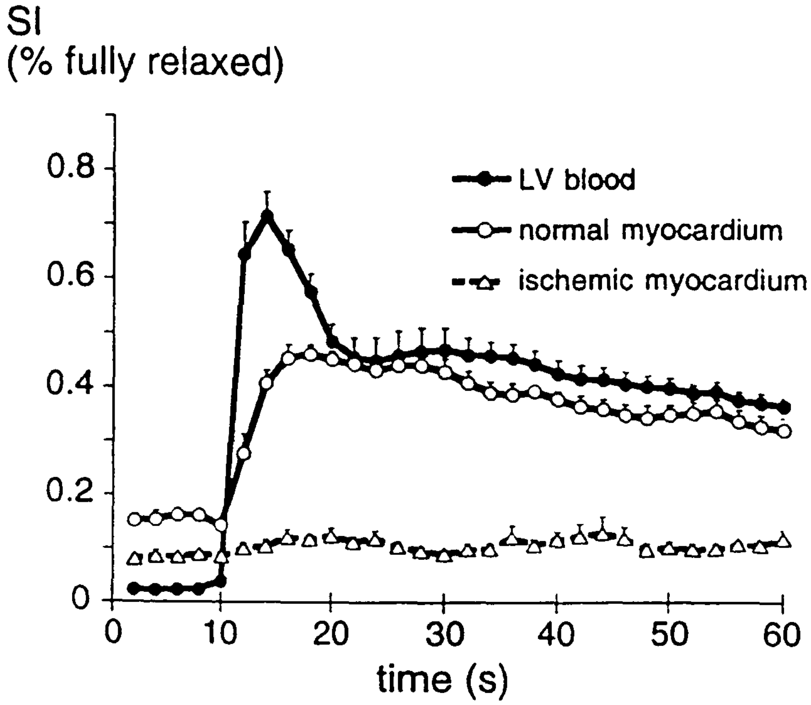
SI
(% fully relaxed)
0.8
0.6
0.4
0.2
0
LV blood
normal myocardium
ischemic myocardium
0
10
20
30
40
50
60
time (s)

FIG. 2D

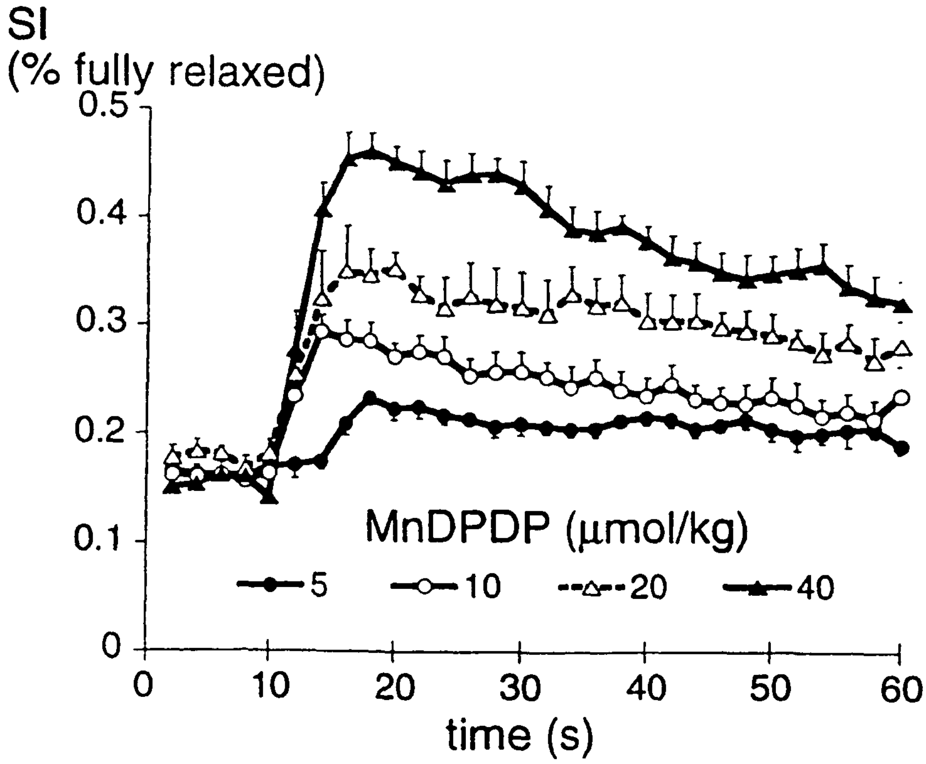
SI
(% fully relaxed)
0.5
0.4
0.3
0.2
0.1
0
MnDPDP (μmol/kg)
5
10
20
40
0
10
20
30
40
50
60
time (s)

FIG. 2E

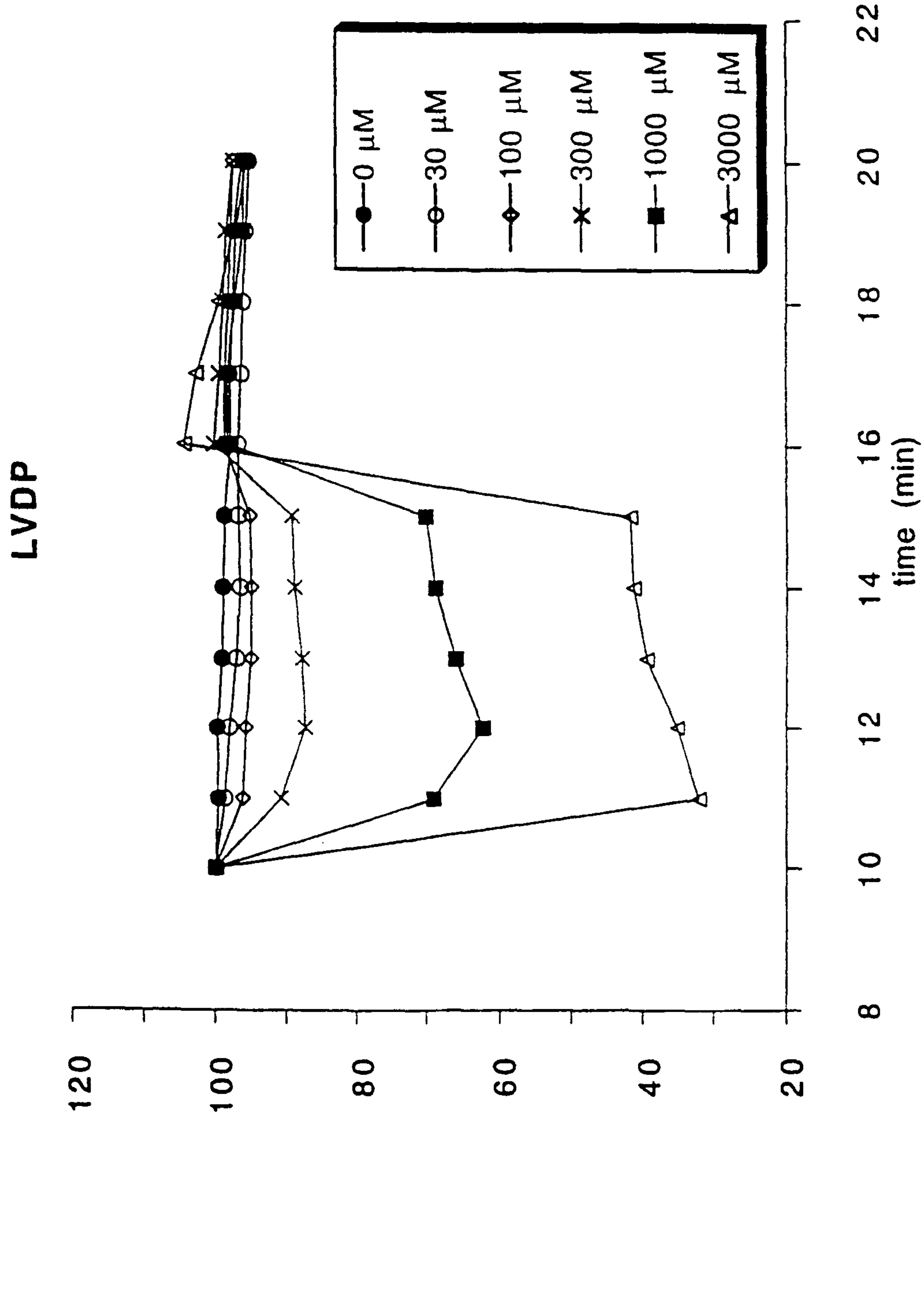
LVDP
120
100
80
60
40
20
8
10
12
14
16
18
20
22
time (min)
0 μM
30 μM
100 μM
300 μM
1000 μM
3000 μM

FIG. 3

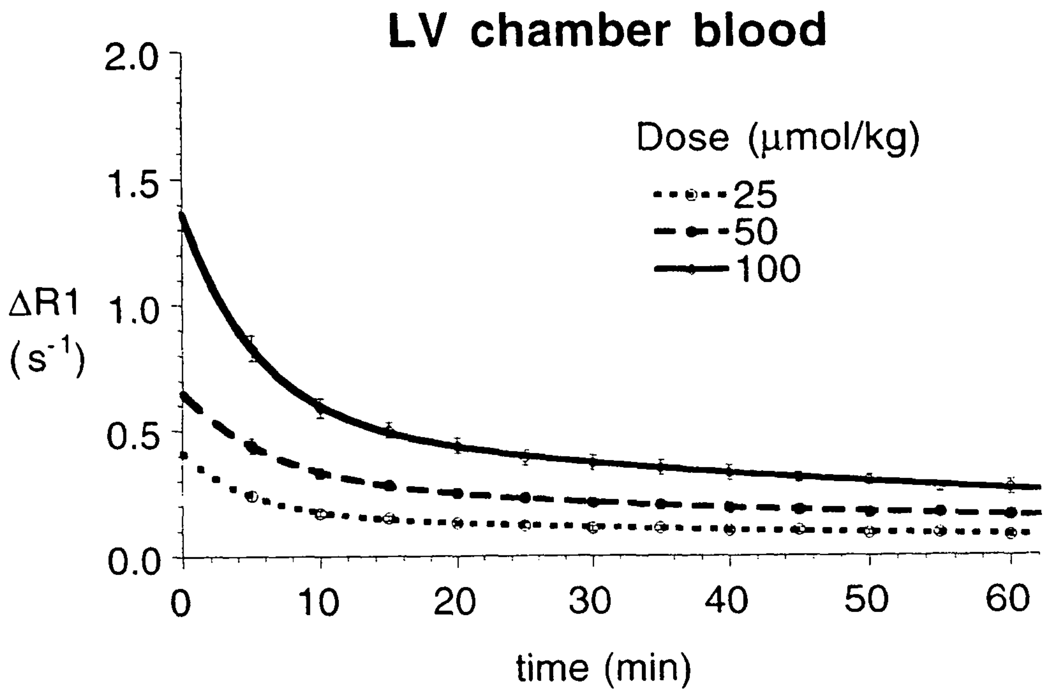
LV chamber blood
2.0
1.5
1.0
0.5
0.0
ΔR1
($s^{-1}$)
Dose (μmol/kg)
25
50
100
0
10
20
30
40
50
60
time (min)

FIG. 4A

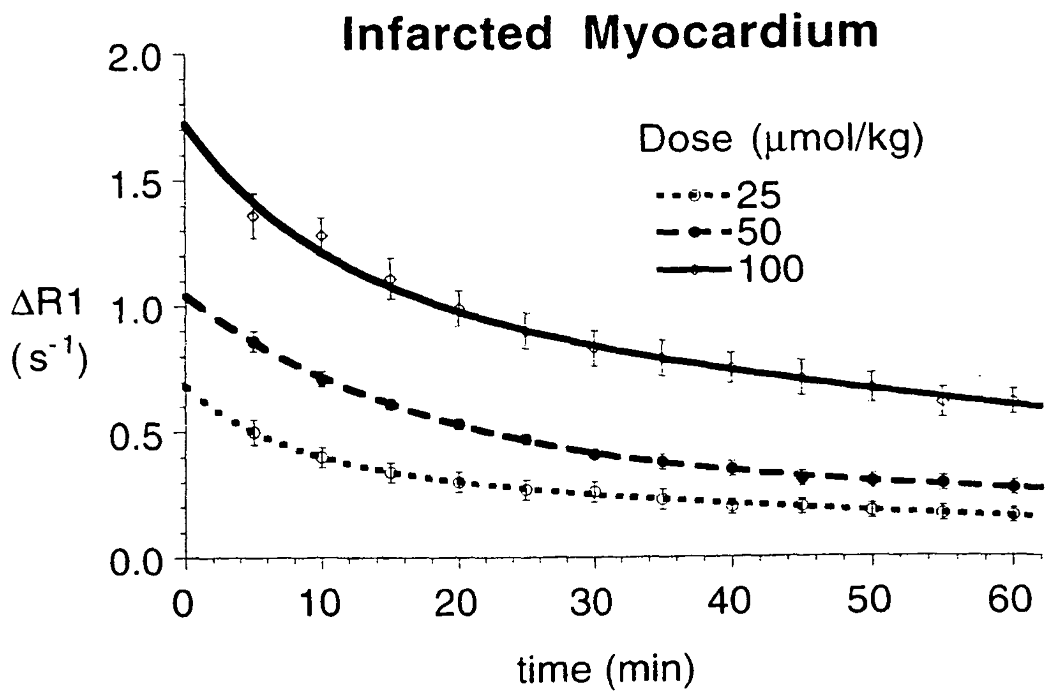
Infarcted Myocardium
2.0
1.5
1.0
0.5
0.0
ΔR1
($s^{-1}$)
Dose (μmol/kg)
25
50
100
0
10
20
30
40
50
60
time (min)

FIG. 4B

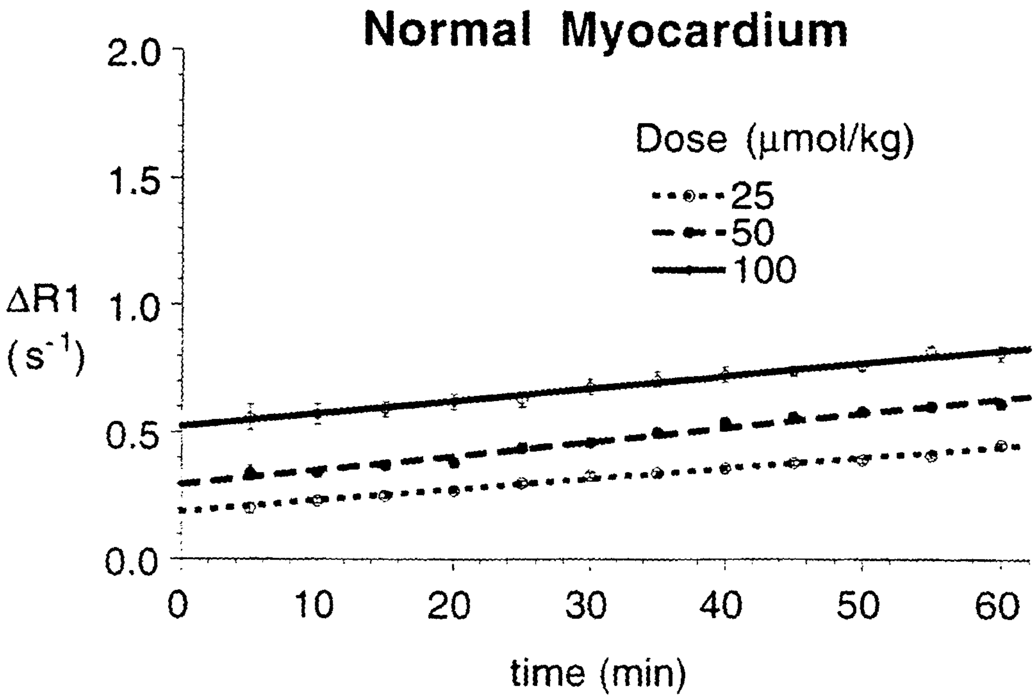
Normal Myocardium
Dose (µmol/kg)
25
50
100
ΔR1
(s-1)
2.0
1.5
1.0
0.5
0.0
0
10
20
30
40
50
60
time (min)

FIG. 4C

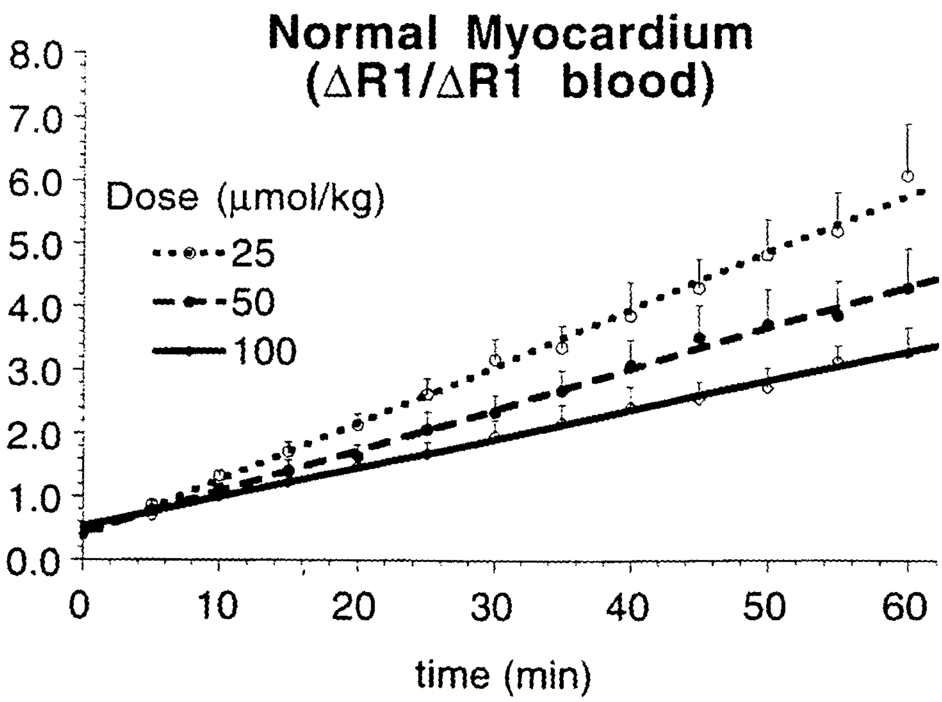
Normal Myocardium
(ΔR1/ΔR1 blood)
Dose (µmol/kg)
25
50
100
8.0
7.0
6.0
5.0
4.0
3.0
2.0
1.0
0.0
0
10
20
30
40
50
60
time (min)

FIG. 5A

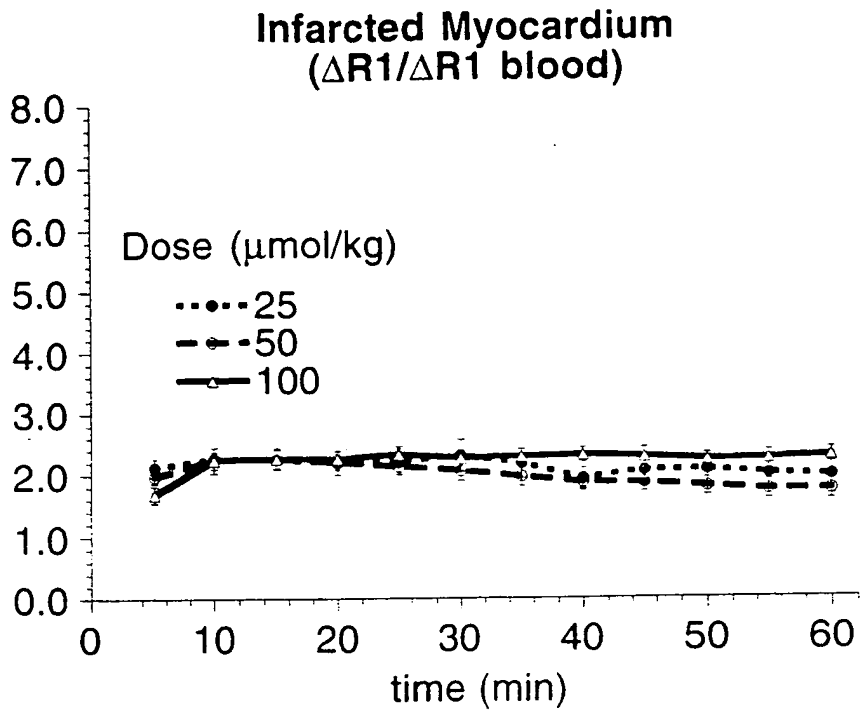
Infarcted Myocardium
(ΔR1/ΔR1 blood)
8.0
7.0
6.0
5.0
4.0
3.0
2.0
1.0
0.0
Dose (μmol/kg)
25
50
100
0
10
20
30
40
50
60
time (min)

FIG. 5B

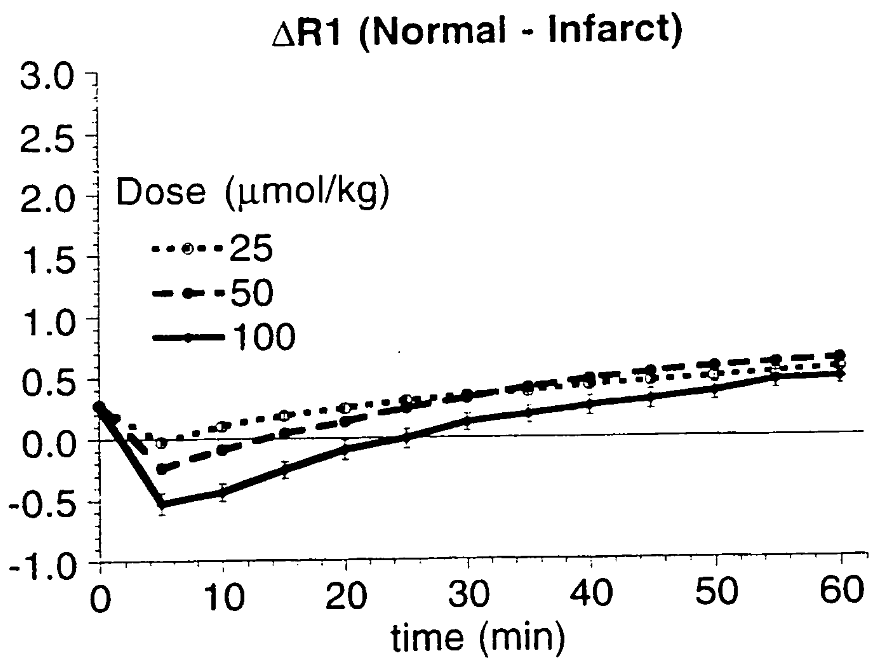
ΔR1 (Normal - Infarct)
3.0
2.5
2.0
1.5
1.0
0.5
0.0
-0.5
-1.0
Dose (μmol/kg)
25
50
100
0
10
20
30
40
50
60
time (min)

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 299795 A
- EP 71564 A
- DE 3401052 A
- EP 203962 A
- EP 436579 A
- EP 290047 A

### Non-patent literature cited in the description


- **POMEROY et al.** *Invest. Radiology,* 1989, vol. 24, 531-536
- **BROWN et al.** *AJR,* 1988, vol. 151, 865-872
- **SAEED et al.** *Radiology,* 1989, vol. 172, 59-64
- **SAEED et al.** *Radiology,* 1990, vol. 175, 633-637
- Remington's Pharmaceutical Sciences. Mack Publishing Co, 1975, 1405-1412 14611487